(19) Europäisches Patentamt
European Patent Office
Office européen des brevets

(11) Publication number: **0 103 265**
**B1**

(12) # EUROPEAN PATENT SPECIFICATION

(45) Date of publication of patent specification: **03.12.86**

(51) Int. Cl.[4]: **C 07 C 69/65,** C 07 C 67/08, C 07 C 67/11, C 07 D 307/04, A 61 K 31/215, A 61 K 31/34

(21) Application number: **83108796.0**

(22) Date of filing: **07.09.83**

(54) **Biphenylylpropionic acid derivative, process for preparing the same and pharmaceutical composition containing the same.**

(30) Priority: **10.09.82 JP 158578/82**
**19.02.83 JP 26559/83**
**06.06.83 JP 101519/83**

(43) Date of publication of application:
**21.03.84 Bulletin 84/12**

(45) Publication of the grant of the patent:
**03.12.86 Bulletin 86/49**

(84) Designated Contracting States:
**AT BE CH DE FR GB IT LI LU NL**

(56) References cited:
**EP-A-0 032 620**
**GB-A-1 359 987**

(73) Proprietor: **Kaken Pharmaceutical Co., Ltd.**
**No. 28-8, Honkomagome 2-chome Bunkyo-ku**
**Tokyo (JP)**

(72) Inventor: **Uchida, Katsuhiro 336, Kashiwaya-cho**
**Yanaginobanbadoori Gojo-agaru Shimogyo-ku**
**Kyoto-shi Kyoto-fu (JP)**
Inventor: **Masumoto, Shozo**
**720, Oaza-Tobaotsu Yasu-cho**
**Yasu-gun Shiga-ken (JP)**
Inventor: **Tohno, Masao**
**2-4, Tsukinowa 3-chome**
**Otsu-shi Shiga-ken (JP)**
Inventor: **Mimura, Mitsuo**
**5-15-2, Ohira 1-chome**
**Otsu-shi Shiga-ken (JP)**
Inventor: **Okumura, Makoto**
**330-5, Imajuku-cho**
**Moriyama-shi Shiga-ken (JP)**
Inventor: **Ichikawa, Kiyonoshin**
**72-2, Yagi-cho**
**Omihachiman-shi Shiga-ken (JP)**
Inventor: **Matsumura, Misako**
**110, Shichiri Ryuo-cho**
**Gamo-gun Shiga-ken (JP)**

(74) Representative: **Türk, Dietmar, Dr. rer. nat. et al**
**Türk, Gille + Hrabal Patentanwälte Bruckner**
**Strasse 20**
**D-4000 Düsseldorf 13 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 103 265 B1

**Description**

The present invention relates to novel biphenylylpropionic acid ester derivatives. More particularly, the present invention relates to biphenylylpropionic acid ester derivatives having the formula (I):

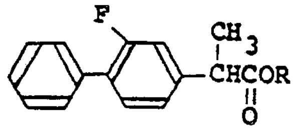

(I)

wherein R is

a) an alkylcarbonyloxyalkyl group or an alkenylcarbonyloxyalkyl group having the formula (II):

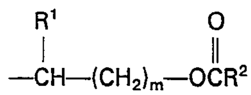

(II)

wherein $R^1$ is hydrogen atom or a lower alkyl group having 1 to 5 carbon atoms, $R^2$ is an alkyl group having 1 to 15 carbon atoms or an alkenyl group having 2 to 8 carbon atoms, and m is 0 or an integer of 1, provided that when $R^1$ is a lower alkyl group, m is 0, or

b) a lactone having the formula (III):

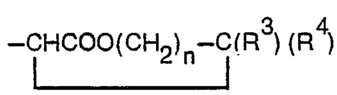

(III)

wherein $R^3$ and $R^4$ are hydrogen atom or a lower alkyl group having 1 to 2 carbon atoms, and n is an integer of 1 or 2, a process for preparing the same and a pharmaceutical composition containing the same as an effective ingredient.

It is known that 2-(2-fluoro-4-biphenylyl)propionic acid (hereinafter referred to as "FP") has strong anti-inflammatory, analgesic and antipyretic activities. However, the formulation form of FP is limited in the form of injection, syrup, or an external preparation such as ophthalmic agent, suppository, cream or plaster because of its irritation. Thus, various modifications are required for a pharmaceutical preparation of FP and the preparation is difficult.

As a result of various studies, the present inventors have now found that a satisfactory drug which has no irritation, an excellent pharmacological effect being several times higher than that of FP and less side effects. That is, the compound (I) of the present invention prepared from FP by esterifying has no irritation. Moreover, the compound (I) is excellent in absorption from mucosa or skin because of its high hydrophobic property. Thus, the pharmacological effect of the compound (I) is quickly appeared and increased. On the other hand, when the compound (I) is formulated in combination with an oleaginous base, the pharmacological effect of the compound (I) is increased and prolonged, and the bioavailability of the compound (I) is increased. Further, the compound (I) is hard to bind with plasma proteins because of its physicochemical properties such as no free polar group and oil. As a result, the tissue distribution and metabolism of the compound (I) after administration are different from those of FP. Accordingly, the concentration of the compound (I) at an inflammation site is increased to show excellent pharmacological effects.

Though a sustained-release agent is generally utilized in order to prolong a pharmacological effect, the compound (I) of the present invention gains a prolongation of a pharmacological effect by advantageously utilizing reaction of metabolic enzyme. More particularly, since the compound (I) in which $R^1$ is a lower alkyl group or R is a lactone having the formula (III) is consisted of diastereomers having two asymmetric carbon atoms in a molecular, there shows a difference of hydrolyzing rate among the diastereomers when the compound (I) is hydrolyzed by metabolic enzymes in tissues. In case of measuring by gas chromatography, for instance, there shows about 5-fold difference in the hydrolyzing rate in plasma. Thus, FP being a parent material of the compound (I) as a physiologically active form is slowly and complementarily released in tissues to prolong an effective blood concentration of FP.

Therefore, the compound (I) is excellent as a drug having no irritation, excellent pharmacological effects, rapid and long-acting, and large safety margin.

It is an object of the present invention to provide novel FP ester derivatives which are useful and characteristic pro-drugs utilizing advantageously enzyme reactions and having excellent anti-inflammatory, analgesic and antipyretic activities, less side effects and high safety.

A further object of the invention is to provide a process for preparing FP ester derivatives.

Another object of the invention is to provide a pharmaceutical composition containing FP ester derivatives as effective ingredients.

These and other objects of the invention will become apparent from the description hereinafter.

In accordance with the present invention, there can be provided a FP ester derivative having the formula (I):

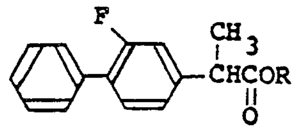

(I)

wherein R is

a) an alkylcarbonyloxyalkyl group or an alkenylcarbonyloxyalkyl group having the formula (II):

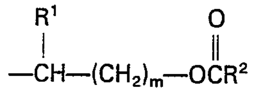

(II)

wherein $R^1$ is hydrogen atom or a lower alkyl group having 1 to 5 carbon atoms, $R^2$ is an alkyl group having 1 to 15 carbon atoms or an alkenyl group having 2 to 8 carbon atoms, and m is 0 or an integer of 1, provided that, when $R^1$ is a lower alkyl group, m is 0, or

b) a lactone having the formula (III):

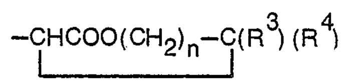

(III)

wherein $R^3$ and $R^4$ are hydrogen atom or a lower alkyl group having 1 to 2 carbon atoms, and n is an integer of 1 or 2.

The preferable substituent group defined as R in the formula (I) is acetoxymethyl, propionyloxymethyl, isobutyryloxymethyl, pivaloyloxymethyl, palmitoyloxymethyl, crotonoyloxymethyl, 3,3-dimethylacryloyloxymethyl, 1-acetoxyethyl, 1-acetoxypropyl, 1-propionyloxyethyl, 1-isobutyryloxyethyl, 1-pivaloyloxyethyl, 1-palmitoyloxyethyl, 1-crotonoyloxyethyl, 1-(3,3-dimethylacryloyloxy)ethyl, 1-(2,4-hexadienoyloxy)ethyl, 2-acetoxyethyl, 2-propionyloxyethyl, 2-crotonoyloxyethyl, 2-(3,3-dimethylacryloyloxy)ethyl, 2-(2,4-hexadienoyloxy)ethyl, 2-(3,7-dimethyl-2,6-octadienoyloxy)ethyl or 3,3-dimethyl-γ-butyrolactone-2-yl group.

Representative compounds among the FP derivatives (I) are as follows:

Compound No. 1: acetoxymethyl 2-(2-fluoro-4-biphenylyl)propionate
Compound No. 2: propionyloxymethyl 2-(2-fluoro-4-biphenylyl)propionate
Compound No. 3: isobutyryloxymethyl 2-(2-fluoro-4-biphenylyl)propionate
Compound No. 4: crotonoyloxymethyl 2-(2-fluoro-4-biphenylyl)propionate
Compound No. 5: 3,3-dimethyl-acryloyloxymethyl 2-(2-fluoro-4-biphenylyl)propionate
Compound No. 6: palmitoyloxymethyl 2-(2-fluoro-4-biphenylyl)propionate
Compound No. 7: pivaloyloxymethyl 2-(2-fluoro-4-biphenylyl)propionate
Compound No. 8: 1-(acetoxy)ethyl 2-(2-fluoro-4-biphenylyl)propionate
Compound No. 9: 1-(propionyloxy)ethyl 2-(2-fluoro-4-biphenylyl)propionate
Compound No. 10: 1-(isobutyryloxy)ethyl 2-(2-fluoro-4-biphenylyl)propionate
Compound No. 11: 1-(pivaloyloxy)ethyl 2-(2-fluoro-4-biphenylyl)propionate
Compound No. 12: 1-(palmitoyloxy)ethyl 2-(2-fluoro-4-biphenylyl)propionate
Compound No. 13: 1-(acetoxy)propyl 2-(2-fluoro-4-biphenylyl)propionate
Compound No. 14: 1-(crotonoyloxy)ethyl 2-(2-fluoro-4-biphenylyl)propionate
Compound No. 15: [1-(3,3-dimethylacryloyloxy)ethyl] 2-(2-fluoro-4-biphenylyl)propionate
Compound No. 16: [1-(2,4-hexadienoyloxy)ethyl] 2-(2-fluoro-4-biphenylyl)propionate
Compound No. 17: 2-(acetoxy)ethyl 2-(2-fluoro-4-biphenylyl)propionate
Compound No. 18: 2-(propionyloxy)ethyl 2-(2-fluoro-4-biphenylyl)propionate
Compound No. 19: 2-(crotonoyloxy)ethyl 2-(2-fluoro-4-biphenylyl)propionate
Compound No. 20: 2-(3,3-dimethylacryloyloxy)ethyl 2-(2-fluoro-4-biphenylyl)propionate
Compound No. 21: 2-(2,4-hexadienoyloxy)ethyl 2-(2-fluoro-4-biphenylyl)propionate
Compound No. 22: 2-(3,7-dimethyl-2,6-octadienoyloxy)ethyl 2-(2-fluoro-4-biphenylyl)propionate
Compound No. 23: 3,3-dimethyl-2-[2-(2-fluoro-4-biphenylyl)propionyloxy]-γ-butyrolactone

The FP derivatives (I) of the present invention are prepared by reacting FP or the salt thereof having the formula (IV):

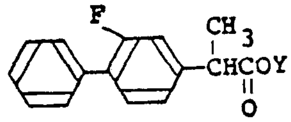

(IV)

wherein Y is hydrogen atom or a metal cation, with a compound having the formula (V):

$$X-\overset{\displaystyle R^1}{\overset{|}{C}H}-(CH_2)_m-O\overset{\displaystyle O}{\overset{\|}{C}}R^2 \qquad (V)$$

wherein $R^1$, $R^2$ and m are as defined above and X is a halogen atom, or a compound having the formula (VI):

$$HO-\underbrace{CHCOO(CH_2)_n-C}(R^3)(R^4) \qquad (VI)$$

wherein $R^3$, $R^4$ and n are as defined above.

The FP derivatives having the formula ($I_0$):

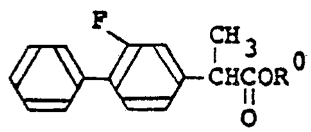

($I_0$)

wherein $R^0$ is an alkylcarbonyloxyalkyl group or an alkenylcarbonyloxyalkyl group having the formula ($II_0$):

$$-CH_2-CH_2-O\overset{\displaystyle O}{\overset{\|}{C}}R^2 \qquad (II_0)$$

wherein $R^2$ is an alkyl group having 1 to 15 carbon atoms or an alkenyl group having 2 to 8 carbon atoms, are also prepared by reaction 2-hydroxyethyl ester of FP having the formula (VII):

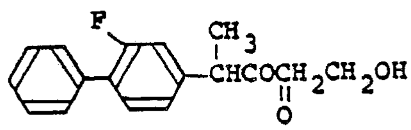

(VII)

with an acid anhydride having the formula (VIII):

$$(R^5\underset{\displaystyle O}{\underset{\|}{C}})_2O \qquad (VIII)$$

wherein $R^5$ is a lower alkyl group having 1 to 2 carbon atoms or an alkenyl group having 2 to 7 carbon atoms, or by reacting a 2-haloethyl ester FP having the formula (IX):

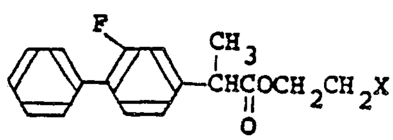

(IX)

wherein X is a as defined above,

with a compound having the formula (X):

$$R^5COOH \qquad (X)$$

wherein $R^5$ is as defined above.

Though the above reactions can be conducted by employing any conventional esterifying reaction, the following method is preferred in point of yield and industrial production.

That is, the esterifying reactions between FP or the salt thereof having the formula (IV) and the compound (V), and the compound (IX) and the compound (X) are generally carried out in an aprotic solvent such as N,N-dimethylformamide, dimethylsulfoxide or hexamethylsulfonyltriamide, or an organic solvent such as acetonitrile, dichloromethane, dichloroethane, chloroform, benzene, ether or tetrahydrofuran, in the presence or absence of an alkali metal carbonate such as sodium carbonate, potassium carbonate, sodium bicarbonate or potassium bicarbonate, an alkali hydroxide such as sodium hydroxide or potassium hydroxide, an alkali metal hydride such as sodium hydride, potassium hydride or lithium hydride, an organic base such as pyridine, triethylamine, N,N-dimethylaniline or tetramethylethylenediamine, an alkali metal iodide such as sodium iodide or potassium iodide, or a phase transfer catalyst such as a crown ether, e.g. 15-crown-5 or 18-crown-6, [2,2,2]-cryptand or [2,2,2]-benzocryptand.

Examples of the salt of the compound (IV) employed in the above reaction are, for instance, metal salts such as silver and copper salt, alkali metal salts such as lithium, sodium and potassium salts. The above-mentioned aprotic solvents may also be employed in combination with ether, tetrahydrofuran, benzene, chloroform, dichloromethane, dichloroethane or acetone.

On the other hand, the esterifying reaction between a FP derivative (IV) and an alcohol derivative (VI) is carried out by dehydration condensation in an organic solvent, under neutral condition and in the absence or presence of a catalyst or a dehydrating agent.

Suitable examples of the organic solvent employed in the above reaction are, for instance, dichloromethane, chloroform, ether, benzene, tetrahydrofuran, and the like. Examples of the dehydrating agent employed in the above reaction are, for instance, N,N-dicyclohexylcarbodiimide, 1-ethyl-3(3-dimethylaminopropyl)carbodiimide (water soluble carbodiimide), their hydrochlorides, and the like. Examples of the catalyst employed in the above reaction are, for instance, pyridine, halogenopyridine, tributylamine, aminopyridine, p-dimethylaminopyridine, and the like. The use of p-dimethylaminopyridine produces a particularly high yield.

As one of the applications of an active esterifying reaction, the desired compound (I) may be prepared by reacting an imidazoate obtained by reacting a FP derivative (IV) with carbonyldiimidazole, with the above alcohol derivative (VI). The desired compound (I) may be also prepared in a high yield by dehydration condensation conducted by using triphenylphosphin or triethyl phosfite and azodicarboxylic acid dialkyl ester.

The amounts of the compounds (V) or (VI), (VIII) and (X) to be used in the above reactions are usually employed in an amount not less than 1.0 mole, preferably 1.0 to 1.5 moles, per mole of the compounds (IV), (VIII) and (IX), respectively.

The reaction temperature is not particularly limited, but the reaction is usually carried out at a temperature of 0° to 120°C. Though the reaction time is varied depending on the reaction conditions such as kinds of solvent or catalyst and temperature, the reaction is usually carried out for several minutes to more than ten hours.

The FP derivatives (I) of the present invention have excellent anti-inflammatory, analgesic and antipyretic activities, and also a high degree of biological hydrolysis. Particularly, 1-(acetoxy)ethyl 2-(2-fluoro-4-biphenylyl)propionate, 1-(propionyloxy)ethyl 2-(2-fluoro-4-biphenylyl)propionate, 1-(crotonoyloxy)ethyl 2(2-fluoro-4-biphenylyl)propionate and 3,3-dimethyl-2-[2-(2-fluoro-4-biphenylyl)propionyloxy]-γ-butyrolactone show excellent pharmaceutical effects. Accordingly, the FP derivatives (I) are very useful as anti-inflammatory, analgesic, antipyretic and anti-rheumatoid agents. They can be formulated in a usual manner into compositions in the form of tablet, capsule, suppository and cream with conventional pharmaceutical carriers. Any conventional carriers used in preparations can be employed in the present invention. Examples of the carrier are, for instance, excipients, binders, lubricants, coloring agents, sterilized water, plant oil, harmless organic solvent, perfumes, emulsifying agents, dispersing agents, and the like.

With respect to the representative compounds of the present invention (the above-mentioned Compound Nos. 1 to 23), the values of $ED_{50}$ (50% effective dose) orally or intravenously in rats by a carrageenan-induced edema inhibitory test, $UD_{50}$ (50% ulcerative dose) orally in rats by a gastric irritation test, hydrolyzing rate in human plasma and $LD_{50}$ (50% lethal dose) orally in mice are shown in Table 1.

TABLE 1

| | Compound No. | $ED_{50}$ mg./kg. p.o. | $ED_{50}$ mg./kg. i.v. | $UD_{50}$ mg./kg. | Hydrolyzing rate (%) | $LD_{50}$ mg./kg. |
|---|---|---|---|---|---|---|
| Compounds of the invention | 1 | 0.5 | 0.08 | 3.6 | 97.5 | 880 |
| | 2 | 0.5 | 0.07 | 2.5 | 94.6 | 750 |
| | 3 | 0.6 | 0.10 | 2.0 | 86.3 | 600 |
| | 4 | 0.6 | 0.08 | 2.0 | 81.4 | 600 |
| | 5 | 0.5 | 0.06 | 2.0 | 64.8 | 900 |
| | 6 | 1.5 | 0.50 | 5.0 | 3.8 | >1000 |
| | 7 | 0.6 | 0.10 | 2.5 | 70.3 | 840 |
| | 8 | 0.5 | 0.05 | 2.5 | 70.7 | 650 |
| | 9 | 0.7 | 0.06 | 3.0 | 56.3 | 800 |
| | 10 | 1.5 | 0.1 | 5.0 | 35.2 | 880 |
| | 11 | 2.5 | 0.6 | 10.0 | 8.3 | >1000 |
| | 12 | >5.0 | >5.0 | >10.0 | 2.0 | >1000 |
| | 13 | 1.5 | 0.1 | 5.0 | 36.0 | 900 |
| | 14 | 0.9 | 0.08 | 4.0 | 42.0 | 800 |
| | 15 | 2.5 | 0.3 | 7.0 | 19.8 | 1000 |
| | 16 | 2.0 | 0.3 | 7.0 | 20.4 | 1000 |
| | 17 | 0.7 | — | 2.0 | 100.9* | 650 |
| | 18 | 0.7 | — | 2.0 | 98.9* | 700 |
| | 19 | 0.9 | — | 3.0 | 99.5* | 800 |
| | 20 | 1.0 | — | 3.0 | 99.8* | 880 |
| | 21 | 0.9 | — | 3.0 | 100.3* | 750 |
| | 22 | 0.9 | — | 3.0 | 101.0* | 750 |
| | 23 | 0.5 | 0.05 | 2.5 | 72.3* | 670 |
| Comparative compounds | acemethacin | 8.3 | — | 17.5 | 4.2 | 18.0 |
| | indomethacin | 5.5 | 1.25 | 5.0 | — | 14.0 |
| | FP | 0.8 | 0.3 | 1.0 | — | 440 |

* values in rat plasma

As is clear from Table 1, the compounds (I) of the present invention have excellent pharmacological effects in comparison with the comparative compounds such as acemethacin, indomethacin and FP. For example, the inhibitory effect on carrageenan-induced edema of the compound (I) is about 8 times that of indomethacin and the same as that of FP. Especially, in the case of intravenously administration, the activity of Compound No. 8 is about 6 times that of FP and about 25 times that of indomethacin. With respect to ulceration which is one of the main side effects of anti-inflammatory agents, the ulceration with

the compound (I) is about one third that of FP. $LD_{50}$ of the compound (I) is 1.5 to 2.0 times higher than that of FP. Thus, the acute toxicity of the compound (I) is considerably reduced. Furthermore, the safety margin of the compound (I) expressed by a ratio of $UD_{50}$ to $ED_{50}$ is about 3 times broader than that of FP.

The effective dose of the compound (I) is about 20 to about 700 mg./day to adult.

The present invention is more particularly described and explained by means of the following Examples, in which all % are by weight unless otherwise noted. In order to illustrate the preparation of (1-chloroethyl)crotonate, (1-chloroethyl)-3,3-dimethylacrylate, (1-chloroethyl)-2,4-hexadienoate, (2-hydroxyethyl)-2-(2-fluoro-4-biphenylyl)propionate and (2-bromoethyl) 2-(2-fluoro-4-biphenylyl)propionate employed as starting materials for preparing the FP derivatives (I) of the invention, the following Reference Examples are also presented.

Reference Example 1

[(1-Chloroethyl)crotonate]

Into a reaction vessel were added 4.8 g. of paraldehyde and 0.14 g. of 96% zinc chloride. To the resulting mixture was added dropwise 10.5 g. (0.1 mole) of crotonoyl chloride with ice-cooling, and then the reaction mixture was reacted with stirring at a room temperature for 2 hours. The resulting reaction mixture was poured into cold water and extracted with 100 ml of ether. The obtained extract was washed successively with a saturated sodium hydrogencarbonate solution and two portions of a saturated sodium chloride solution. After drying the organic layer with anhydrous magnesium sulfate, the solvent was distilled away under reduced pressure to give a yellow liquor. The product was further distilled under reduced pressure (bp: 87 to 88°C./37 mmHg) in an atmosphere of nitrogen gas to give 8.44 g. (yield: 56.8%) of the desired compound being a clear solution.

GC—MS (20 eV) m/e: 148 ($m^+$), 150 (M+2), 113, 86, 69 (base peak), 41 and 27.

Reference Example 2

[(1-Chloroethyl)-3,3-dimethylacrylate]

The procedure of Reference Example 1 was repeated except that 6.8 g of paraldehyde, 0.2 g (2 mmoles) of 96% zinc chloride and 18.5 g (0.156 mole) of 3,3-dimethylacryloyl chloride were employed, to give 21.54 g. (yield: 85%) of the desired compound being a clear solution.

Boiling point: 86° to 87°C./24 mmHg.

GC—MS (20 eV) m/e: 162 ($m^+$), 164 (M+2), 126, 100, 83 (base peak), 63, 55 and 29.

Reference Example 3

[(1-Chloroethyl)-2,4-hexadienoate]

A mixture of 5.6 g. (50 mmoles) of sorbic acid and 7.8 g. (65 mmoles) of thionyl chloride was stirred at a room temperature for one hour, and then reacted at 50°C. for 30 minutes with stirring to complete the reaction. The end point of the reaction was judged by cessation of $SO_2$ gas production and disappearance (dissolution) of sorbic acid. The resulting reaction mixture was cooled with ice, and then 0.27 g. (2 mmoles) of 96% zinc chloride was added thereto. After adding dropwise 2.2 g. of paraldehyde at 0°C., the reaction was carried out at a room temperature for one hour. The resulting reaction mixture was treated and purified in the same manner as in Reference Example 1 to give 5.43 g. (yield: 64.2%) of the desired compound being a clear solution.

Boiling point: 111° to 112°C./18 mmHg.

GC—MS (20 eV) m/e: 175 ($m^+$), 176 (M+2), 111, 97, 95 (base peak), 67 and 41.

Reference Example 4

[(2-Hydroxyethyl) 2-(2-fluoro-4-biphenylyl)propionate]

In 45 ml. of anhydrous dimethylformamide (hereinafter referred to as "DMF") was dissolved 12.2 g. (50 mmoles) of FP. To the resulting solution was added 6.9 g. (50 mmles) of anhydrous potassium carbonate. After adding dropwise 6.25 g. (50 mmoles) of ethylene bromohydrin with ice-cooling, the reaction was carried out at 60° to 70°C. for 15 hours with stirring. After cooling the reaction mixture with ice, the resulting inorganic material was filtered off, and then the solvent was distilled away under reduced pressure. To the resulting residue was added 50 ml. of diethyl ether, and then the resulting mixture was washed successively with water, 10% sodium carbonate solution and a saturated sodium chloride solution, and the organic layer was dried with magnesium sulfate. The solvent was distilled away under reduced pressure to give 13.8 g. of clear oily material. The resulting oily material was further purified by chromatography of silica gel (Kiesel gel 60 F made by Merck & Co., Inc.; Developing solvent: (1) dichloromethane (2) dichloromethane:ether = 9:1 (3) dichloromethane:ether = 8:2) to give 9.97 g. (yield: 69.2%) of white crystal of the desired compound having a melting point of 73° to 75°C.

Mass spectrum (20 eV, Direct) m/e:

288 ($m^+$), 244, 199 (base peak), 184, 178 and 45.

Reference Example 5

[(2-Bromoethyl) 2-(2-fluoro-4-biphenylyl)propionate]

In 40 ml. of dichloromethane was dissolved 12.2 g. (50 mmoles) of FP. To the resulting solution were added 6.25 g. (50 mmoles) of ethylene bromohydrin and 0.3 g. (2.5 mmoles) of p-dimethylaminopyridine.

After adding dropwise 10.3 g. (50 mmoles) of dicyclohexylcarbodiimide dissolved in dichloromethane, the reaction was carried out at a room temperature for 30 minutes with stirring. After cooling the reaction mixture with ice, the insoluble material was filtered off, and then the organic layer was washed successively with 0.1N hydrochloride, saturated sodium hydrogencarbonate solution and saturated sodium chloride solution, and dried with anhydrous magnesium sulfate. After distilling away the solvent under reduced pressure, the precipitated dicyclohexylurea was filtered off. The resulting yellowish oily material was purified in the same manner as in Reference Example 4 to give 15.6 g. (yield: 89%) of the desired compound being a clear oily material.

Mass spectrum (20 eV, Direct) m/e:

351 ($m^+$), 353 (M+2), 244, 199 (base peak), 184, 178, 108 and 110.

Example 1

[Acetoxymethyl 2-(2-fluoro-4-biphenylyl)propionate (Compound No. 1)]

In 100 ml. of anhydrous DMF was dissolved 7.32 g. (30 mmoles) of FP. To the resulting solution was added 2.1 g. (15 mmoles) of anhydrous potassium carbonate with ice-cooling, and then the resulting mixture was stirred for one hour. After adding dropwise 3.3 g. (30 mmoles) of acetoxymethyl chloride purified by distillation at 0° to 5°C. for 10 minutes, the reaction was carried out with stirring at a room temperature for 2 hours. After cooling the resulting reaction mixture with ice and filtering off the inorganic materials, the solvent was distilled away under reduced pressure. To the obtained residue was added 150 ml. of diethyl ether, and then the residue was washed successively with water, 10% of sodium carbonate solution and a saturated sodium chloride solution. The obtained organic layer was dried with anhydrous magnesium sulfate, and then the solvent was distilled away under reduced pressure to give 8.24 g. (yield: 86.9%) of the desired compound being an oily material.

The product was further distilled under reduced pressure in an atmosphere of nitrogen gas to give 6.55 g. (yield: 69.1%) of an oily material having a boiling point of 195° to 197°C./0.4 mmHg.

Elementary analysis for $C_{18}H_{17}C_4F$ (MW: 316):

Calcd. (%): C 68.35 H 5.38

Found (%): C 68.42 H 5.51.

Nuclear magnetic resonance spectrum (in $CCl_4$, TMS, δ (ppm):

1.49 (d, 3H, >CH—C$\underline{H}_3$), 2.00 (s, 3H, —OCO—C$\underline{H}_3$), 3.74 (q, 1H, $CH_3$—C$\underline{H}$<), 5.71 (s, 2H, —O—C$\underline{H}_2$—O—), 7.03 to 7.56 (m, 8H, aromatic H).

Mass spectrum (20 eV, Direct) m/e:

316 ($M^{+}$), 226 ([M—$OCH_2OAc]^+$), 199 (base beak, [M—$CO_2CH_2OAc]^+$), 73 [—$CH_2OAc]^+$ and 43 [—$COCH_3]^+$.

Infrared absorption spectrum (ν $cm.^{-1}$):

3100 to 2850 (aromatic, alkyl ν C—H), 1760 (ν COOR), 1625 to 1420 (aromatic, ν C=C) and 1370 (ν $COCH_3$).

Refractive index: $n_D^{26}$ = 1.5488.

Ultraviolet absorption: λ max = 248 nm.

Example 2

[Propionyloxymethyl 2-(2-fluoro-4-biphenylyl)propionate (Compound No. 2)]

In 100 ml. of anhydrous DMF was dissolved 7.32 g. (30 mmoles) of FP. To the resulting solution was added 2.1 g. (15 mmoles) of anhydrous potassium carbonate with ice-cooling, and then the reaction mixture was stirred for one hour. After adding dropwise 4.78 g. (39 mmoles) of propionyloxymethyl chloride purified by distillation with ice-cooling, the reaction was carried out with stirring at a room temperature for 2 hours, and then at 60° to 70°C. for one hour. After completion of the reaction, the reaction mixture was cooled, and then the inorganic material was filtered off. After distilling away the solvent under reduced pressure, 150 ml. of dimethyl ether was added to the residue, and then the organic layer was washed successively with water, 10% sodium carbonate solution and a saturated sodium chloride solution. After drying the obtained organic layer with anhydrous magnesium sulfate, the solvent was distilled away under reduced pressure. The resulting product was further distilled under reduced pressure in an atmosphere of nitrogen gas to give 7.35 g. (yield: 74.2%) of the desired compound being a colorless oily material having a boiling point of 195° to 196°C./0.8 mmHg.

Elementary analysis for $C_{19}H_{19}O_4F$ (MW: 330):

Calcd. (%): C 69.09 H 5.76

Found (%): C 69.33 H 5.98

Nuclear magnetic resonance spectrum (in $CCl_4$, TMS), δ (ppm):

1.08 (t, 3H, —$CH_2$—C$\underline{H}_3$), 1.52 (d, 3H, C$\underline{H}_3$—CH), 2.26 (q, 2H, —C$\underline{H}_2$—$CH_3$), 3.72 (q, 1H, $CH_3$—C$\underline{H}$), 5.69 (s, 2H, —O—C$\underline{H}_2$—O—) and 7.06 to 7.55 (m, 8H, aromatic H)

Mass spectrum (20 eV, Direct) m/e:

330 ($M^{+}$),

226 ($[M-OCH_2O\overset{\overset{O}{\|}}{C}Et]^+$), 199 ($[M-CO_2CH_2O\overset{\overset{O}{\|}}{C}Et]^+$), 87 ($[-CH_2-O\overset{\overset{O}{\|}}{C}Et]^+$) and 57 (base peak, $[-\overset{\overset{O}{\|}}{C}Et]^+$).

Infrared absorption spectrum (ν $cm.^{-1}$):

3100 to 2850 (aromatic, alkyl ν C—H), 1760 (ν R—COO—R) and 1625 to 1420 (aromatic ν C=C).

Refractive index: $n_D^{26}$ = 1.5431.

Ultraviolet absorption: λ max = 248 nm.

Example 3

[Isobutyryloxymethyl 2-(2-fluoro-4-biphenylyl)propionate (Compound No. 3)]

The procedure of Example 2 was repeated except that 4.08 g. (30 mmoles) of isobutylyloxymethyl chloride was employed instead of propionyloxymethyl chloride, to give 7.29 g. (yield: 70.6%) of the desired compound being an oily material having a boiling point of 188° to 190°C./0.4 mmHg.

Elementary analysis for $C_{20}H_{21}O_4F$ (MW: 344)

Calcd. (%): C 69.77 H 6.10

Found (%): C 70.03 H 6.08.

Nuclear magnetic resonance spectrum (in $CCl_4$, TMS), δ (ppm):

0.98 to 1.20 (m, 6H, $-CH\langle{}^{CH_3}_{CH_3}$), 1.50 (d, 3H, $\underline{CH_3}-CH<$), 2.46 (m, 1H, $-C\underline{H}\langle{}^{CH_3}_{CH_3}$,

3.72 (q, 1H, $CH_3-C\underline{H}$), 5.69 (s, 2H, —O—$CH_2$—O—) and 7.00 to 7.60 (m, 8H, aromatic H).

Mass spectrum (20 eV, Direct) m/e:

344 ($M^+$), 243 ($[M-CH_2O\overset{\overset{O}{\|}}{C}CH(CH_3)_2]^+$), 226 ($[M-OCH_2O\overset{\overset{O}{\|}}{C}CH(CH_3)_2]^+$),

199 (base peak, $[M-CO_2CH_2O\overset{\overset{O}{\|}}{C}CH(CH_3)_2]^+$), 101 ($[-CH_2O\overset{\overset{O}{\|}}{C}CH(CH_3)_2]^+$),

71 ($[-COCH(CH_3)_2]^+$) and 43 ($[-CH(CH_3)_2]^+$).

Infrared absorption spectrum (ν $cm.^{-1}$):

3100 to 2850 (aromatic, alkyl ν C—H), 1755 (ν COO—R) and 1625 to 1420 (aromatic ν C=C).

Refractive index: $n_D^{26}$ = 1.5409.

Example 4

[Crotonoyloxymethyl 2-(2-fluoro-4-biphenylyl)propionate (Compound No. 4)]

The procedure of Example 2 was repeated except that 4 g. (30 mmoles) of crotonoyloxymethyl chloride instead of propionyloxymethyl chloride was employed, to give 5.57 g. (yield: 54.3%) of the desired compound being an oily material having a boiling point of 217° to 219°C./0.4 mmHg.

Elementary analysis for $C_{20}H_{19}O_4F$ (MW: 342)

Calcd. (%): C 70.18 H 5.55

Found (%): C 70.46 H 5.79.

Nuclear magnetic resonance spectrum (in $CCl_4$, TMS), δ (ppm):

1.52 (d, 3H, $\underline{CH_3}-CH<$), 1.68 to 1.95 (m, 3H, olefinic $\underline{CH_3}$), 3.73 (q, 1H, $CH_3-\underline{CH}<$), 5.67 to 5.94 (m, 1H, olefinic H), 5.75 (s, 2H, —O—$\underline{CH_2}$—O—), 6.80 to 6.98 (m, 1H, olefinic H) and 7.00 to 7.52 (m, 8H, aromatic H).

Mass spectrum (20 eV, Direct) m/e:

342 ($M^+$), 226 ($[M-OCH_2O\overset{\overset{O}{\|}}{C}CH=CHCH_3]^+$), 199 ($[M-CO_2CH_2O\overset{\overset{O}{\|}}{C}CH=CHCH_3]^+$) and

69 (base peak, $[-COCH=CHCH_3]^+$).

Infrared absorption spectrum (ν $cm.^{-1}$):

3100 to 2850 (aromatic, alkyl ν C—H), 1740 (ν COO—R) and

1658 (ν 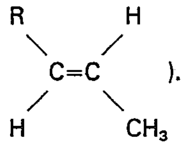 ).

Refractive index: $n_D^{26}$ = 1.5525.
Ultraviolet absorption: λ max = 248 nm.

Example 5

[3,3-Dimethylacryloyloxymethyl 2-(2-fluoro-4-biphenylyl)propionate (Compound No. 5)]

The procedure of Example 2 was repeated except that 5.8 g. (39 mmoles) of 3,3-dimethylacryloyloxymethyl chloride instead of propionyloxymethyl chloride was employed, to give 6.57 g. (yield: 61.5%) of the desired compound being an oily material having a boiling point of 210° to 214°C./0.5 mmHg.

Elementary analysis for $C_{21}H_{21}O_4F$ (MW: 356):
Calcd. (%): C 70.79 H 5.90
Found (%): C 71.12 H 6.25.

Nuclear magnetic resonance spectrum (in $CCl_4$, TMS), δ (ppm):
1.52 (d, 3H, $C\underline{H}_3$—CH<), 1.94 (s, 3H, olefinic $C\underline{H}_3$), 2.10 (s, 3H, olefinic $C\underline{H}_3$), 3.70 (q, 1H, $CH_3$—C$\underline{H}$<), 5.55 to 5.69 (m, 1H, olefinic H), 5.72 (s, 2H, —O—$C\underline{H}_2$—O—) and 7.00 to 7.51 (m, 8H, aromatic H).

Mass spectrum (20 eV, Direct) m/e:

356 ($M^+$), 226 ([M—$OCH_2$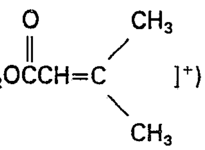 ]$^+$) 199 ([M—$CO_2CH_2$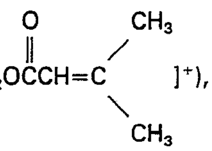 ]$^+$),

83 (base peak, 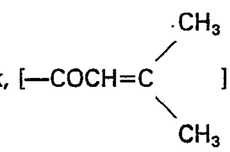 ]$^+$) and 55 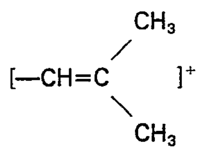 ]$^+$

Infrared absorption spectrum (ν $cm.^{-1}$):
3100 to 2850 (aromatic, alkyl ν C—H), 1745 (ν COO—R),

1645 (olefinic ν 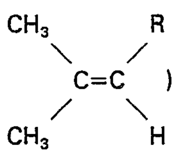 ) and 1625 to 1420 (aromatic ν C=C).

Refractive index: $n_D^{26}$ = 1.5535.

Example 6

[Palmitoyloxymethyl 2-(2-fluoro-4-biphenylyl)propionate (Compound No. 6)]

The procedure of Example 2 was repeated except that an ether solution of 11.9 g. (39 mmoles) of palmitoyloxymethyl chloride instead of propionyloxymethyl chloride was employed, to give 9.7 g. (yield: 73.1%) of white crystals of the crude desired compound. The resulting product was purified by centrifugal liquid chromatography (carrier: KT 2106 made by Fuji Gel Co., Ltd.; Eluent: cyclohexane:dichloromethane = 1:1) to give 7.3 g. (yield: 47.5%) of white crystals of the desired compound having a melting point of 45.5° to 48°C.

Elementary analysis for $C_{32}H_{45}O_4F$ (MW: 512):
Calcd. (%): C 75.0 H 8.79
Found (%): C 75.34 H 9.04.

Nuclear magnetic resonance spectrum (in $CCl_4$, TMS), δ (ppm):
0.88 (t, 3H, —$CH_2C\underline{H}_3$), 1.11 to 1.42 (m, 26H, alkyl H), 1.51 (d, 3H, $C\underline{H}_3$—CH<), 2.23 (t, 2H, —OCO—$C\underline{H}_2$—$CH_2$—), 3.71 (q, 1H, $CH_3$—C$\underline{H}$<), 5.65 (s, 2H, —O—$C\underline{H}_2$—O—) and 7.00 to 7.67 (m, 8H, aromatic H).

Mass spectrum (20 eV, Direct) m/e:

512 ($M^{+\cdot}$), 244 ($[M—CH_2O\overset{\overset{O}{\|}}{C}(CH_2)_{14}CH_3]^+$), 239 $[—\overset{\overset{O}{\|}}{C}O(CH_2)_{14}CH_3]^+$ and

199 (base peak, $[M—CO_2CH_2O\overset{\overset{O}{\|}}{C}(CH_2)_{14}CH_3]^+$)

Infrared absorption spectrum (ν $cm.^{-1}$):

3100 to 2800 (aromatic, alkyl ν C—H), 1755 (ν R COO—R) and 1625 to 1420 (aromatic ν C=C)

Example 7

[Pivaloyloxymethyl 2-(2-fluoro-4-biphenylyl)propionate (Compound No. 7)]

The procedure of Example 2 was repeated except that 4.51 g. (30 mmoles) of pivaloyloxymethyl chloride instead of propionyloxymethyl chloride was employed to give 7.73 g. (yield: 72.0%) of the desired compound being an oily material having a boiling point of 191° to 194°C./0.4 mmHg.

Elementary analysis for $C_{21}H_{23}FO_4$ (MW: 358):

Calcd. (%): C 70.45 H 6.49

Found (%): C 69.73 H 6.88.

Nuclear magnetic resonance spectrum (in $CCl_4$, TMS), δ (ppm):

1.10 (s, 9H, $—C(C\underline{H}_3)_3$], 1.50 (d, 3H, $C\underline{H}_3—CH<$), 3.72 (q, 1H, $CH_3—C\underline{H}<$), 5.69 (s, 2H, $—O—C\underline{H}_2—O—$) and 7.00 to 7.60 (m, 8H, aromatic H).

Mass spectrum (20 eV, Direct) m/e:

358 ($M^{+\cdot}$), 243 ($[M—CH_2O\overset{\overset{O}{\|}}{C}C(CH_3)_3]^+$), 227 ($[M—OCH_2O\overset{\overset{O}{\|}}{C}C(CH_3)_3]^+$),

199 (base peak, ($[M—CO_2CH_2O\overset{\overset{O}{\|}}{C}C(CH_3)_3]^+$), 115 $[—CH_2O\overset{\overset{O}{\|}}{C}C(CH_3)_3]^+$),

85 $[—COC(CH_3)_3]^+$ and 57 $[—C(CH_3)_3]^+$.

Infrared absorption spectrum (ν $cm.^{-1}$):

3100 to 2850 (aromatic, alkyl ν C—H), 1755 (ν COOR) and 1625—1420 (aromatic ν C=C).

Refractive index: $n_D^{26}$ = 1.5398.

Ultraviolet absorption: λ max = 248 nm.

Example 8

[Acetoxymethyl 2-(2-fluoro-4-biphenylyl)propionate (Compound No. 1)]

In 150 ml. of anhydrous DMF was dissolved 2.8 g. of potassium salt of FP. To the resulting solution was added 1.2 g. of acetoxymethyl chloride, and the reaction mixture was stirred at a room temperature for one hour. The resulting reaction mixture was treated and purified in the same manner as in Example 1 to give 2.56 g. (yield: 81%) of the desired compound. The physicochemical properties of the obtained compound were consistent with those obtained in Example 1.

Example 9

[1-(Acetoxy)ethyl 2-(2-fluoro-4-biphenylyl)propionate (Compound No. 8)]

In 20 ml. of anhydrous DMF was dissolved 2.44 g. (10 mmoles) of FP. To the resulting solution was added 1.38 g. (10 mmoles) of anhydrous potassium carbonate, and then the reaction mixture was stirred at a room temperature for 30 minutes. After adding dropwise 1.45 g. (12 mmoles) of (1-chloroethyl)acetate purified by distillation at 0° to 5°C., the reaction was carried out at 60° to 70°C. for one hour with stirring.

After completion of the reaction, the reaction mixture was cooled with ice, and then the inorganic material was filtered off. After distilling away the solvent under reduced pressure, 50 ml. of diethyl ether was added to the obtained residue. The resulting mixture was treated in the same manner as in Example 2 to give 2.97 g. (crude yield: 90%) of light yellowish oily material.

The resulting product was further distilled under reduced pressure in an atmosphere of nitrogen gas to give 2.4 g. (yield: 74%) of the desired compound being a clear oily material having a boiling point of 173° to 174°C./0.8 mmHg.

Elementary analysis for $C_{19}H_{19}FO_4$ (MW: 330):

Calcd. (%): C 69.09 H 5.76

Found (%): C 69.16 H 5.89.

Nuclear magnetic resonance spectrum (in $CCl_4$, TMS), δ (ppm):

1.34 (d, 3H, 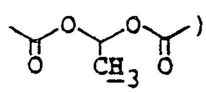), 1.45 (d, 3H, 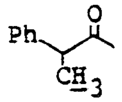),

1.95 (2s, 3H, $-O\overset{O}{\overset{\|}{C}}-C\underline{H}_3$), 3.60 (q, 1H, $Ph-\overset{|}{C}\underline{H}-CO_2-$),

6.68 to 6.89 (2q, 1H, $-COO-\overset{|}{C}\underline{H}-O\overset{O}{\overset{\|}{C}}-$) and 6.90 to 7.43 (m, 8H, aromatic H).

Mass spectrum (20 eV, Direct) m/e:

330 ($M^{+}$), 244 $(M-CH(CH_3)O\overset{O}{\overset{\|}{C}}CH_3)^+$, 226 $(M-OCH(CH_3)O\overset{O}{\overset{\|}{C}}CH_3)^+$, 199 [base peak, 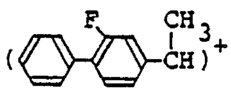], 87 $[CH(CH_3)O\overset{O}{\overset{\|}{C}}CH_3]^+$, 72 $[\overset{|}{C}HO\overset{O}{\overset{\|}{C}}CH_3]^+$ and 43 $[COCH_3]^+$.

Infrared absorption spectrum (ν cm.$^{-1}$):
3060 to 2875 (aromatic, alkyl, ν C—H), 1770 (ν C=O ester) and 1630 (aromatic ν C=C).
Refractive index: $n_D^{24.5} = 1.5353$.
Ultraviolet absorption: λ max = 247 nm.

## Example 10

[1-(Propionyloxy)ethyl 2-(2-fluoro-4-biphenylyl)propionate (Compound No. 9)]

The procedure of Example 9 was repeated except that 1.30 g. (10 mmoles) of (1-chloroethyl)propionate instead of (1-chloroethyl)acetate was employed to give 2.5 g. (yield: 73%) of the desired compound being a clear oily material.

Elementary analysis for $C_{20}H_{21}FO_4$ (MW: 344):
Calcd. (%): C 69.77 H 6.10
Found (%): C 69.97 H 6.12.

Nuclear magnetic resonance spectrum (in $CCl_4$, TMS), δ (ppm):
1.02 and 1.10 (2t, 3H, $-COCH_2C\underline{H}_3$), 1.34 (d, 3H, 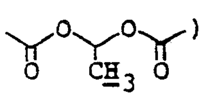), 1.45 (d, 3H, 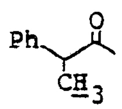), 2.22 and 2.31 (2q, 2H, $-COC\underline{H}_2CH_3$),

3.66 (q, 1H, $Ph-C\underline{H}-\overset{O}{\overset{\|}{C}}-$), 6.60 to 6.78 (m, 1H, $-COO\overset{|}{C}\underline{H}O\overset{O}{\overset{\|}{C}}-$) and 6.79 to 7.32 (m, 8H, aromatic H).

Mass spectrum (20 eV, Direct) m/e:

344 ($M^{+}$), 266 $[M-OCH(CH_3)O\overset{O}{\overset{\|}{C}}-C_2H_5]^+$, 199 [base peak, 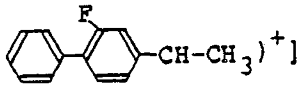], 101 $[CH(CH_3)O\overset{O}{\overset{\|}{C}}-C_2H_5]^+$, 57 $[COC_2H_5]^+$ and 29 $[C_2H_5]^+$.

Infrared absorption spectrum (ν cm.$^{-1}$):
3060 to 2875 cm.$^{-1}$ (aromatic, alkyl ν C—H), 1760 (ν C=O ester) and 1630 (aromatic ν C=C).
Refractive index: $n_D^{24.5} = 1.5331$.
Ultraviolet absorption λ max = 247 nm.
Boiling point: 188° to 191°C./0.4 mmHg.

## Example 11

[1-(Isobutyryloxyethyl) 2-(2-fluoro-4-biphenylyl)propionate (Compound No. 10)]

The procedure of Example 9 was repeated except that 1.50 g. (10 mmoles) of 1-chloroethyl isobutylate instead of 1-chloroethyl acetate was employed to give 2.72 g. (yield: 76%) of the desired compound being a clear oily material.

Elementary analysis for $C_{21}H_{23}FO_4$ (MW: 358):
Calcd. (%): C 70.39 H 6.42
Found (%): C 70.57 H 6.44.

Nuclear magnetic resonance spectrum (in $CCl_4$, TMS), δ (ppm):
1.14 (2d, 6H —$CH(C\underline{H}_3)_2$, 1.39 to 1.54 (m, 6H,

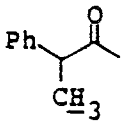 , 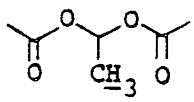 ), 2.46 (m, 1H, —$C\underline{H}\genfrac{}{}{0pt}{}{\diagup CH_3}{\diagdown CH_3}$ ),

3.64 and 3.67 (2q, 1H, Ph—$C\underline{H}$—$\overset{\overset{O}{\|}}{C}$—), 6.61 to 6.31 (m, 1H, —COOCHO$\overset{\overset{O}{\|}}{C}$—),

6.95 to 7.43 (m, 8H, aromatic H).

Mass spectrum (20 eV, Direct) m/e:

358 ($M^{+\cdot}$), 226 [M—OCH($CH_3$)O$\overset{\overset{O}{\|}}{C}$—CH$(CH_3)_2]^+$, 199 [base peak,

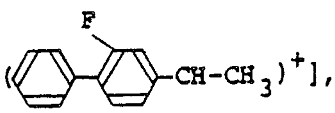 , 131 [CH($CH_3$)O$\overset{\overset{O}{\|}}{C}$—CH$(CH_3)_2]^+$,

71 [COCH$(CH_3)_2]^+$, 43 [CH$(CH_3)_2]^+$.

Infrared absorption spectrum (ν $cm.^{-1}$):
3060 to 2875 (aromatic, alkyl ν C—H), 1760 (ν C=O ester) and 1630 (aromatic ν C=C).

Refractive index: $N_D^{24.5}$ = 1.5267.

Ultraviolet absorption: λmax = 247 nm.

Boiling point: 160° to 165°C./0.5 mmHg.

## Example 12

[1-(Pivaloyloxy)ethyl 2-(2-fluoro-4-biphenylyl)propionate (Compound No. 11)]

The procedure of Example 9 was repeated except that 2.1 g. (13 mmoles) of (1-chloroethyl) 2,2-dimethyl propionate instead of (1-chloroethyl)acetate was employed to give 2.1 g. (yield: 56%) of the desired compound being a clear oily material.

Elementary analysis for $C_{22}H_{25}FO_4$ (MW: 372):
Calcd. (%): C 70.97 H 6.72
Found (%): C 71.18 H 6.68.

Nuclear magnetic resonance spectrum (in $CCl_4$, TMS), δ (ppm):
1.14 (2s, 9H, —$C(C\underline{H}_3)_3$, 1.39 to 1.54 (2d, 6H,

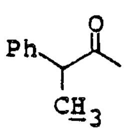 , 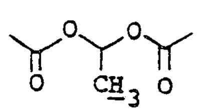 ), 3.64 and 3.67 (2q, 1H, Ph—$\overset{|}{C}\underline{H}$—$\overset{\overset{O}{\|}}{C}$—),

6.61 to 6.81 (m, 1H, —COOC$\underline{H}$O$\overset{\overset{O}{\|}}{C}$—),

6.95 to 7.43 (m, 8H, aromatic H).

Mass spectrum (20 eV, Direct) m/e:

372 (M $^{+}$), 226 [M—OCH($CH_3$)OC(=O)—C($CH_3$)$_3$], 199 [base peak,

( 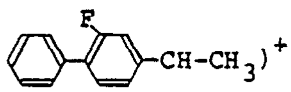 )$^{+}$], 129 [CH($CH_3$)OC(=O)—C($CH_3$)$_3$]$^{+}$,

85 [COC($CH_3$)$_3$]$^{+}$, 57 [C($CH_3$)$_3$]$^{+}$

Infrared absorption spectrum (ν $cm.^{-1}$):

3060 to 2875 (aromatic, alkyl ν C—H), 1760 (ν C=O ester), 1630 (aromatic ν C=C).

Refractive index: $n_D^{24.5}$ = 1.5203.

Ultraviolet absorption: λ max = 247 nm.

Boiling point: 166° to 172°C./0.55 mmHg.

Example 13

[1-(Palmitoyloxy)ethyl 2-(2-fluoro-4-biphenylyl)propionate (Compound NO. 12)]

The procedure of Example 9 was repeated except that 3.18 g. (10 mmoles) of (1-chloroethyl)palmitate instead of (1-chloroethyl)acetate was employed to give 3.1 g. of clear oil material. The obtained product was purified by chromatography of silica gel [Kiesel gel 60 made by Merck & Co., Inc. (60 g.); Eluent: (1) cyclohexane, (2) cyclohexane:dichloromethane = 8:2, (3) cyclohexane:dichloromethane = 6:4] to give 2.67 g. (yield: 50.7%) of the desired compound being a clear oily material.

Elementary analysis for $C_{33}H_{47}FO_4$ (MW: 326):

Calcd. (%): C 75.29 H 8.94

Found (%): C 75.51 H 8.97.

Nuclear magnetic resonance spectrum (in $CCl_4$, TMS), δ (ppm):

0.83 to 1.72 (m, 35H, alkyl H),

2.18 (t, 2H, —OC(=O)—$C\underline{H}_2$—$CH_2$—), 3.69 (q, 1H, Ph—C$\underline{H}$—C(=O)—), 6.68 to 6.86 (m, 1H, —COOC$\underline{H}$OC(=O)—) and

6.98 to 7.52 (m, 8H, aromatic H).

Mass spectrum (20 eV, Direct) m/e:

526 (M $^{+}$), 239 [base peak, (CO($CH_2$)$_{14}$$CH_3$)$^{+}$] and

199 [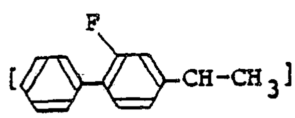]$^{+}$,

Infrared absorption spectrum (ν $cm.^{-1}$):

3075 to 2875 (aromatic, alkyl ν C—H), 1770 (ν C=O ester), 1630 (aromatic ν C=C).

Refractive index: $n_D^{24.5}$ = 1.5028.

Ultraviolet absorption: λ max = 247 nm.

Example 14

[1-(Crotonoyloxy)ethyl 2-(2-fluoro-4-biphenylyl)propionate (Compound No. 14)]

The procedure of Example 9 was repeated except that 1.38 g. (10 mmoles) of (1-chloroethyl)cronate purified by distillation instead of (1-chloroethyl) acetate was employed to give 1.6 g. (yield: 44.9%) of the desired compound being a clear oily material.

Elementary analysis for $C_{21}H_{21}FO_4$ (MW: 356):

Calcd. (%): C 70.79 H 5.90

Found (%): C 71.00 H 5.93.

Nuclear magnetic resonance spectrum (in $CCl_4$, TMS), δ (ppm):

1.40 (d, 3H, 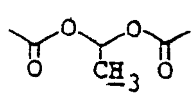), 1.50 (d, 3H, 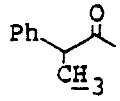),

1.73 to 1.87 (m, 3H, —CH=CH—C$\underline{H}_3$), 3.66 (q, 1H, Ph—CH—C(=O)—),

5.58 to 5.86 (m, 1H, $-C\underline{H}=CH-CH_3$), 6.72 to 6.98 (m, 2H, $-\overset{O}{\overset{\|}{C}}O-\overset{|}{C}\underline{H}O\overset{O}{\overset{\|}{C}}$, $-CH=C\underline{H}-CH_3$), 7.02 to 7.40 (m, 8H, aromatic H).

Mass spectrum (20 eV, Direct) m/e:

356 ($M^{+}$), 226 $[M-OCH(CH_3)O\overset{O}{\overset{\|}{C}}-CH=CH-CH_3]^+$, 199 $[M-CO_2CH(CH_3)O\overset{O}{\overset{\|}{C}}-CH=CH-CH_3]^+$, 113 $[CH(CH_3)O\overset{O}{\overset{\|}{C}}-CH=CH-CH_3]^+$, 69 [base peak, $(COCH=CH-CH_3)^+$] and 41 $[CH=CH-CH_3]^+$.

Infrared absorption spectrum (ν $cm.^{-1}$)(
3100 to 2900 (aromatic, alkyl ν C—H), 1750 (ν C=O ester) and 1665 (ν C=C di-substituted olefine).

Refractive index: $n_D^{24.5}$ = 1.5397.

Ultraviolet absorption: λ max = 247 nm. and 206 nm.

Boiling point: 195° to 197°C./0.3 mmHg.

Example 15

[[1-(3,3-dimethylacryloyloxy)ethyl] 2-(2-fluror-4-biphenylyl)propionate (Compound No. 15)]

The procedure of Example 9 was repeated except that 2.43 g. (15 mmoles) of (1-chloroethyl)-3,3-dimethylacrylate purified by distillation instead of (1-chloroethyl)acetate was employed to give 3.19 g. (yield: 85.1%) of the desired compound being a clear oily material.

Elementary analysis for $C_{22}H_{23}FO_4$ (MW: 370):
Calcd. (%): C 71.35 H 6.22
Found (%): C 71.14 H 6.21.

Nuclear magnetic resonance spectrum (in $CCl_4$, TMS), δ (ppm):

1.48 and 1.38 (d, 6H, 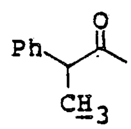 , 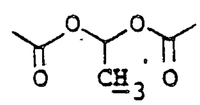 ),

1.85 (d, 3H, $-CH=C<^{C\underline{H}_3}_{CH_3}$ ), 2.15 (d, 3H, $-CH=C<^{CH_3}_{C\underline{H}_3}$ ),

3.66 (q, 1H, $Ph-\overset{|}{C}\underline{H}-COO$), 5.46 to 5.60 (m, 1H, olefinic H), 6.69 to 6.89 (2q, 1H, $-COO\overset{|}{C}\underline{H}-OCO$) and 6.95 to 7.38 (m, 8H, aromatic H).

Mass spectrum (20 eV, Direct) m/e:

370 ($M^{+}$), 226 $[M-OCH(CH_3)O\overset{O}{\overset{\|}{C}}-CH=C(CH_3)_2]^+$, 199 $[M-CO_2CH(CH_3)O\overset{O}{\overset{\|}{C}}-CH=C(CH_3)_2]^+$, 127 $[CH(CH_3)O\overset{O}{\overset{\|}{C}}-CH=C(CH_3)_2]^+$, 83 [base peak, $COCH=C(CH_3)_2^+$] and 55 $[CH=C(CH_3)_2]^+$.

Infrared absorption spectrum (ν $cm.^{-1}$):
3075 to 2900 (aromatic, alkyl ν C—H), 1750 (ν C=O ester), 1660 (ν C=C, tri-substituted olefine).

Refractive index: $n_D^{24.5}$ = 1.5423.

Ultraviolet absorption: λ max = 224 nm. and 206 nm.

Boiling point: 194°C./0.6 mmHg.

Example 16

[1-(2,4-Hexadienoyloxy)ethyl] 2-(2-fluoro-4-biphenylyl)propionate (Compound No. 16)]

The procedure of Example 13 was repeated except that 2.26 g. (13 mmoles) of (1-chloroethyl)-2,4-hexadienoate purified by distillation instead of (1-chloroethyl)palmitate was employed to give 3.17 g. (yield: 83.0%) of the desired compound being a clear oily material.

Elementary analysis for $C_{23}H_{23}FO_4$ (MW: 382)

Calcd. (%): C 72.25 H 6.02

Found (%): C 72.47 H 6.04.

Nuclear magnetic resonance spectrum (in $CCl_4$, TMS), δ (ppm):

1.40 to 1.55 (m, 6H, 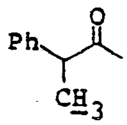 , 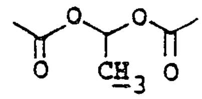 ),

1.77 to 1.89 (m, 3H, —CH=CH—C<u>H</u>$_3$), 3.69 (q, 1H, Ph—C<u>H</u>—$CO_2$),

5.54 to 5.79 (2d, 2H, olefinic H), 6.03 to 6.20 (m, 1H, olefinic H),

6.75 to 6,93 (m, 2H, olefinic H, —COOC<u>H</u>OC(=O)—),

7.00 to 7.46 (m, 8H, aromatic H).

Mass spectrum (20 eV, Direct) m/e:

382 (M$^{+}$), 226 [M—OCH($CH_3$)OC(=O)—CH=CH—CH=CH—$CH_3$]$^2$,

199 [M—$CO_2$CH($CH_3$)OC(=O)—CH=CH—CH=CH—$CH_3$]$^2$,

95 [base peak, (COCH=CH—CH=CH—$CH_3$)$^+$] and 67 [CH=CH—CH=CH—$CH_3$]$^+$.

Infrared absorption spectrum (ν $cm.^{-1}$):

3075 to 2875 (aromatic, alkyl ν C—H), 1755 (ν C=O ester), 1652 (olefinic ν C=C).

Refractive index: $n_D^{24.5}$ = 1.5612.

Ultraviolet absorption: λ max = 254 nm.

Example 17

[1-(Acetoxy)propyl 2-(2-fluoro-4-biphenyl)propionate (Compound No. 13)]

The procedure of Example 13 was repeated except that 1.4 g. (10 mmoles) of (1-chloropropyl)acetate purified by distillation instead of (1-chloroethyl) palmitate was employed to give 1.2 g. (yield: 34.8%) of the desired compound being a blown oily material.

Elementary analysis for $C_{20}H_{21}FO_4$ (MW: 344):

Calcd. (%): C 69.77 H 6.10

Found (%): C 69.99 H 6.12.

Nuclear magnetic resonance spectrum (in $CCl_4$, TMS), δ (ppm):

0.72 to 1.00 (m, 3H, —CHCH$_2$C<u>H</u>$_3$),

1.50 (d, 3H, 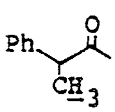 ), 1.60 to 1.83 (m, 2H, —CHC<u>H</u>$_2$CH$_3$), 1.95 (2s, 3H, —OCOC<u>H</u>$_3$),

3.66 (q, 1H, Ph—C<u>H</u>—COO), 6.49 to 6.66 (m, 1H, —COOC<u>H</u>OC(=O)—),

6.95 to 7.35 (m, 8H, aromatic H).

Mass spectrum (20 eV, Direct) m/e:

344 ($M^{+}$), 199 [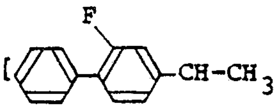]$^{+}$, 101 $[CH(C_2H_5)O\overset{O}{\overset{\|}{C}}—CH_3)^+$ and 43 $[COCH_3]^+$.

Infrared absorption spectrum (ν $cm.^{-1}$):
3080 to 2900 (aromatic, alkyl ν C—H), 1770 (ν C=O ester), 1632 (aromatic ν C=C).
Refractive index: $n_D^{24.5}$ = 1.5310.
Ultraviolet absorption: λ max = 247 nm.

Example 18

[1-(Acetoxy)ethyl 2-(2-fluoro-4-biphenylyl)propionate (Compound No. 8)]

Into 140 ml. of anhydrous DMF was suspended 2.82 g. (10 mmoles) of potassium salt of FP. To the resulting supsension was added dropwise 1.47 g. (12 mmoles) of (1-chloroethyl)acetate dissolved in 10 ml. of anhydrous DMF while keeping the reaction mixture at a temperature of 60°C., and then the resulting reaction mixture was stirred at 80°C. for one hour. After completion of the reaction, the resulting reaction mixture was treated and purified in the same manner as in Example 9 to give 2.5 g. (yield: 75%) of the desired compound being a clear oily material.

The physicochemical properties of the compound were consistent with those obtained in Example 9.

Example 19

[1-(Propionyloxy)ethyl 2-(2-fluoro-4-biphenylyl)propionate (Compound No. 9)]

To 30 ml. of dehydrated acetonitrile were added 0.84 g. (3 mmoles) of potassium salt of FP, 0.3 g. (3 mmoles) of triethylamine and 0.5 g. (3 mmoles) of potassium iodide.

To the resulting reaction mixture was added 0.47 g. (4 mmoles) of (1-chloroethyl)propionate at a room temperature with stirring, and then the reaction was carried out at 60°C. for 5 hours. After cooling, the resulting residue was filtered off, and then 100 ml. of ether was added to the filtrate. The resulting organic layer was washed successively with 10% sodium carbonate solution, water and a saturated sodium chloride aqueous solution by a conventional method, and then dried with magnesium sulfate. After distilling away the solvent, the obtained oily material was distilled under reduced pressure to give 0.84 g. (yield: 83%) of the desired compound. The physicochemical properties of the compound were consistent with those obtained in Example 10.

Example 20

[1-(Pivaloyl)ethyl 2-(2-fluoro-4-biphenylyl)propionate (Compound No. 11)]

To 40 ml. of dehydrated acetonitrile was added 20 mg. of 18-crown-6, and then the resulting reaction mixture was stirred at room temperature for 30 minutes. To the resulting reaction mixture were added 0.56 g. (2 mmoles) of potassium salt of FP and then 0.49 g. (3 mmoles) of (1-chloroethyl)-2,2-dimethylpropionate, and the reaction was carred out at 50°C. for 8 hours. After cooling and added 100 ml. of ether, the resulting reaction mixture was treated and purified in the same manner as in Example 12 to give 0.37 g. (yield: 50%) of the desired compound. The physicochemical properties of the compound were consistent with those obtained in Example 12.

Example 21

[1-(Acetoxy)ethyl 2-(2-fluoro-4-biphenylyl)proprionate (Compound No. 8)]

In 25 ml. of acetonitrile was dissolved 2.44 g. (10 mmoles) of FP. To the resulting solution was added 1.38 g (10 mmoles) of anhydrous potassium carbonate, and then the reaction mixture was stirred at a room temperature for 30 minutes. After adding dropwise 2.4 g. (20 mmoles) of (1-chloroethyl)acetate purified by distillation, the reaction was carried out at a room temperature for 4 hours, and then at 40°C. for one hour with stirring. After cooling, the resulting residue was filtered off, and 100 ml. of ether was added to the filtrate. The resultling organic layer was washed successively with 10% sodium carbonate solution, water and a saturated sodium chloride aqueous solution, and then dried with magnesium sulfate. After distilling away the solvent, the obtained oily material was distilled under reduced pressure to give 2.3 g. (yield: 71%) of the desired compound. The physicochemical properties of the compound were consistent with those obtained in Example 9.

Example 22

[1-(Acetoxy)ethyl 2-(2-fluoro-4-biphenylyl)propionate (Compound No. 8)]

The procedure of Example 9 was repeated except that 1.67 g. (10 mmoles) of (1-bromoethyl)acetate instead of (1-chloroethyl)acetate was employed to give 2.56 g. (yield: 81%) of the desired compound being a clear oily material. The physicochemical properties of the compound were consistent with those obtained in Example 9.

## Example 23

[2-(Acetoxy)ethyl 2-(2-fluoro-4-biphenylyl)propionate (Compound No. 17]

In 25 ml. of anhydrous dichloromethane was dissolved 2.44 g. (10 mmoles) of FP. To the resulting solution were added 1.04 g. (10 mmoles) of (2-acetoxy)ethyl alcohol and 0.13 g. (1 mmole) of p-dimethylaminopyridine with stirring at 0°C. After adding dropwise 2.3 g. (11 mmoles) of dicyclohexylcarbodiimide dissolved in anhydrous dichloromethane, the reaction was carried out at room temperature for one hour. After ice-cooling, the insoluble material was filtered off, and then the organic layer was washed successively with 0.1 N hydrochloride, a saturated sodium hydrogencarbonate solution and a saturated sodium chloride solution, and dried with anhydrous magnesium sulfate. After distilling away the solvent under reduced pressure, the resulting clear oily material was distilled under reduced pressure for purification. The fractions of distillate having a boiling point of 234° to 238°C./mmHg were collected to give 2.6 g. (yield: 80%) of the desired compound being a clear oily material.

Elementary analysis for $C_{19}H_{19}FO_4$ (MW: 330.4):

Calcd. (%): C 69.07 H 5.75

Found (%): C 69.28 H 5.77.

Nuclear magnetic resonance spectrum (in $CCl_4$, TMS), δ (ppm):

$$1.55\ (d, 3H, \overset{|}{C}H{-}C\underline{H_3}),\ 1.96\ )s, 3H, {-}O\overset{\overset{O}{\|}}{C}C\underline{H_3}),$$

$$3.74\ (q, 1H, \overset{|}{C}\underline{H}{-}CH_3),\ 4.20\ (s, 4H, {-}\overset{\overset{O}{\|}}{C}OC\underline{H_2}C\underline{H_2}O\overset{\overset{O}{\|}}{C}{-}),$$

6.90 to 7.42 (m, 3H, aromatic proton).

Mass spectrum (20 eV, Direct) m/e:

$$330\ (M^{+}),\ 226,\ 199\ [\text{base peak}, (M{-}\overset{\overset{O}{\|}}{C}OCH_2CH_2O\overset{\overset{O}{\|}}{C}CH_3)^{+}],$$

$$87\ [{-}CH_2CH_2O\overset{\overset{O}{\|}}{C}CH_3]^{+}\ \text{and}\ 43\ [{-}COCH_3]^{+}.$$

Infrared absorption spectrum (ν $cm.^{-1}$):

3100 to 2850 (aromatic, alkyl ν C—H), 1760 (ester ν C=O), 1625 to 1420 (aromatic ν C=C).

Refractive index: $n_D^{27} = 1.5427$.

## Example 24

[2-(Propionyloxy)ethyl 2-(2-fluoro-4-biphenylyl)propionate (Compound No. 18)]

In 25 ml. of anhydrous DMF was dissolved 2.44 g. (10 mmoles) of FP. To the resulting solution was added (1.38 g. (10 mmoles) of anhydrous potassium carbonate, and the reaction mixture was stirred at a room temperature for one hour. After adding dropwise 1.77 g. (13 mmoles) of (2-propionyloxy)ethyl chloride, the reaction was carried out at 40° to 50°C. for one hour with stirring. After ice-cooling, the inorganic material was filtered off, and then the solvent was distilled away under reduced pressure. After adding 50 ml. of diethyl ether to the resulting residue, the mixture was washed successively with water, 10% sodium carbonate solution and a saturated sodium chloride solution, and then the organic layer was dried with anhydrous magnesium sulfate. After distilling away the solvent under reduced pressure, the resulting clear oily material was distilled under reduced pressure for purification. The fractions of distillate having a boiling point of 225°C. to 228°C./0.5 mmHg were collected to give 2.55 g. (yield: 74%) of the desired compound bieng a clear oily material.

Elementary analysis for $C_{20}H_{21}FO_4$ (MW: 344.4):

Calcd. (%): C 69.69 H 6.10

Found (%): C 69.90 H 6.12.

Nuclear magnetic spectrum (in $CCl_4$, TMS), δ (ppm):

$$1.08\ (t, 3H, {-}CH_2C\underline{H_3}),\ 1.52\ (d, 3H, {-}\overset{|}{C}H{-}C\underline{H_3}),\ 2.26\ (q, 2H, {-}C\underline{H_2}CH_3),$$

$$2.75\ (q, 1H, {-}\overset{|}{C}\underline{H}{-}CH_3),\ 4.22\ (s, 4H, {-}\overset{\overset{O}{\|}}{C}OC\underline{H_2}C\underline{H_2}O\overset{\overset{O}{\|}}{C}{-}),$$

6.90 to 7.42 (m, 8H, aromatic proton).

Mass spectrum (20 eV, Direct) m/e:

$$344\ (M^{+}),\ 226,\ 199\ [\text{base peak}\ (M-\overset{O}{\overset{\|}{C}}OCH_2CH_2O\overset{O}{\overset{\|}{C}}C_2H_5)^+],$$

$$101\ [-CH_2CH_2O\overset{O}{\overset{\|}{C}}C_2H_5]^+\ \text{and}\ 57\ [-COC_2H_5]^+.$$

Infrared absorption spectrum (ν $cm.^{-1}$):

3100 to 2850 (aromatic alkyl ν C—H), 1760 (ester ν C=O), 1625 to 1420 (aromatic ν C=C).

Refractive index: $n_D^{27} = 1.5397$.

Example 25

[2-(Crotonoyloxy)ethyl 2-(2-fluoro-4-biphenylyl)propionate (Compound No. 19)]

The procedure of Example 24 was repeated except that 2.89 g. (15 mmoles) of (2-crotonoyloxy)ethyl bromide instead of (2-propionyloxy)ethyl chloride was employed to give 3.39 g. (yield: 63.4%) of the desired compound being a clear oily material having a boiling point of 205° to 215°C./1.5 mmHg.

Elementary analysis for $C_{21}H_{21}FO_4$ (MW: 356.4):

Calcd. (%): C 70.77 H 5.89

Found (%): C 70.98 H 5.91.

Nuclear magnetic resonance (in $CCl_4$, TMS), δ (ppm):

$$1.40\ (d,\ 3H,\ -\overset{|}{C}H-C\underline{H}_3),\ 1.72\ (dd,\ 3H,\ \text{olefinic}\ -C\underline{H}_3),$$

$$3.57\ (q,\ 1H,\ -\overset{|}{C}\underline{H}-CH_3),\ 4.06\ (s,\ 4H,\ -COC\underline{H}_2C\underline{H}_2O\overset{O}{\overset{\|}{C}}-),$$

5.54, 5.51 (dd, 1H, olefinic proton), 6.44 to 7.33 (m, 9H, aromatic, olefinic proton).

Mass spectrum (20 eV, Direct) m/e:

$$356\ (M^{+}),\ 226,\ 199\ [\text{base peak},\ (M-\overset{O}{\overset{\|}{C}}OCH_2CH_2O\overset{O}{\overset{\|}{C}}CH{=}CHCH_3)^+],$$

$$113\ [-CH_2CH_2O\overset{O}{\overset{\|}{C}}CH{=}CHCH_3]^+,\ 69\ [-COCH{=}CHCH_3]^+.$$

Infrared absorption spectrum (δ $cm.^{-1}$):

3100 to 2850 (aromatic alkyl ν C—H), 1720 to 1750 (ester ν C=O), 1665 (olefinic ν C=C).

Refractive index: $n_D^{27} = 1.5461$.

Example 26

[2-(3,3-Dimethylacryloyloxy)ethyl 2-(2-fluoro-4-biphenylyl)propionate (Compound 20)]

The procedure of Example 24 was repeated except that 3.10 g. (15 mmoles) of 2-(3,3-dimethylacryloyloxy)ethylbromide instead of (2-propionyloxy)ethyl chloride, was employed to give 3.31 g. (yield: 59.6%) of the desired compound being a clear oily material having a boiling point of 225° to 228°C./0.8 mmHg.

Elementary analysis for $C_{22}H_{23}FO_4$ (MW: 370.5):

Calcd. (%): C 71.32 H 6.21

Found (%): C 71.53 H 6.24.

Nuclear magnetic resonance spectrum (in $CCl_4$, TMS), δ (ppm):

$$1.44\ (d,\ 3H,\ -\overset{|}{C}-C\underline{H}_3),$$

$$1.72\ (s,\ 3H,\ \text{olefinic}\ -C\underline{H}_3),\ 2.03\ (s,\ 3H,\ \text{olefinic}\ -C\underline{H}_3),\ 3.62\ (q,\ 1H,\ -C\underline{H}-CH_3),$$

$$4.08,\ (s,\ 4H\ -\overset{O}{\overset{\|}{C}}OCH_2CH_2O\overset{O}{\overset{\|}{C}}-),$$

5.40 (m, 2H, olefinic proton) and 6.82 to 7.52 (m, 8H, aromatic proton).

Mass spectrum (20 eV, Direct) m/e:

$$370\ (M^{+}),\ 226,\ 199\ [\text{base peak},\ [M-\overset{O}{\overset{\|}{C}}OCH_2CH_2O\overset{O}{\overset{\|}{C}}CH=C(CH_3)_2]^{+}],$$

$$127\ [-CH_2CH_2O\overset{O}{\overset{\|}{C}}CH=C(CH_3)_2]^{+},$$

83 $[-COCH=C(CH_3)_2]^{+}$ and 55 $[-CH=C(CH_3)_2]^{+}$.

Infrared absorption spectrum (ν cm.$^{-1}$):

3100 to 2850 (aromatic alkyl ν C—H), 1745, 1725 (ester ν C=O) and 1655.

Refractive index: $n_D^{27} = 1.5431$.

Example 27

[2-(3,7-Dimethyl-2,6-octadienoyloxy)ethyll 2-(2-fluoro-4-biphenylyl)propionate (Compound No. 22)]

The procedure of Example 24 was repeated except that 1.68 g. (10 mmoles) of 3,7-dimethyl-2,6-octadienoic acid, 10 ml. of anhydrous DMF, 1.38 g. (10 mmoles) of anhydrous potassium carbonate and 3.51 g. (10 mmoles) of 2-bromoethyl 2-(2-fluoro-4-biphenylyl)propionate were employed. The resulting residue was purified by chromatography of silica gel (Kiesel gel 60 F (90 g.); Developing solvent: a mixed solvent of dichloromethane and cycohexane) to give 3.1 g. (yield: 83.7% of the desired compound being a clear oily material having a boiling point of not less than 250°C./mmHg.

Elementary analysis for $C_{27}H_{31}FO_4$ (MW: 438.6):

Calcd. (%): C 73.94 H 7.07

Found (%): C 74.16 H 7.06.

Nuclear magnetic resonance spectrum (in $CCl_4$, TMS), δ (ppm):

$$1.44\ (d,\ sH,\ Ph-\overset{|}{C}H-C\underline{H}_3),$$

1.46, 1.55 (ss, 6H, olefinic $-CH_3 \times 2$), 1.96 (m, 7H, olefinic $-C\underline{H}_3$, olefinic $-C\underline{H}_2-C\underline{H}_2-$),

$$3.96\ (s,\ 4H,\ -\overset{O}{\overset{\|}{C}}O-CH_2CH_2-O\overset{O}{\overset{\|}{C}}-),$$

4.66 to 4.92 (m, 1H, olefinic proton), 5.24 (s, 1H, olefinic proton) and 6.64 to 7.18 (m, 3H, aromatic proton).

Mass spectrum (20 eV, Direct) m/e:

$$438\ (M^{+}),\ 226,\ [\text{base peak}\ [M-\overset{O}{\overset{\|}{C}}OCH_2CH_2O\overset{O}{\overset{\|}{C}}CH=\overset{CH_3}{\overset{|}{C}}-CH_2CH_2-CH=C(CH_3)_2]^{+}],$$

$$195\ [-CH_2CH_2O\overset{O}{\overset{\|}{C}}CH=C(CH_3)CH_2CH_2CH=C(CH_3)_2]^{+},$$

151 $[-COCH=C(CH_3)CH_2CH_2CH=C(CH_3)_2]^{+}$ and 69 $[-CH_2CH=C(CH_3)_2]^{+}$.

Infrared absorption spectrum (ν cm.$^{-1}$):

3100 to 2850 (aromatic alkyl ν C—H), 1745, 1730 (ester ν C=O) and 1650 (olefinic ν C=C).

Refractive index: $n_D^{27} = 1.5435$.

Example 28

[2-(2,4-Hexadienoyloxy)ethyl 2-(2-fluoro-4-biphenylyl)propionate (Compound No. 21)]

To 200 ml. of anhydrous acetonitrile were added 0.1 g. of 18-crown-6, 1.5 g. (20 mmoles) of potassium sorbate and 3.5 g. (10 mmoles) of (2-bromoethyl) 2-(2-fluoro-4-biphenylyl)propionate, and then the resulting reaction mixture was stirred at 50°C. for 8 hours. The resulting reaction mixture was treated and purified by conventional methods in the same manner as in Example 27 to give 3.5 g. (yield: 91.6 %) of the desired compound being a white crystal having a melting point of 59° to 61°C.

Elementary analysis for $C_{23}H_{23}FO_4$ (MV: 382.3):

Calcd. (%): C 72.26 H 6.02

Found (%): C 72.40 H 6.04.

Nuclear magnetic resonance spectrum (in $CCl_4$, TMS), δ (ppm):

1.44 (d, 3H, Ph—CH(—)—$C\underline{H}_3$), 1.71 (d, 3H, olefinic —$CH_3$),

3.48 (q, 1H, Ph—$C\underline{H}$(—)—$CH_3$), 4.00 (s, 4H, —CO(=O)—$C\underline{H}_2C\underline{H}_2$—OC(=O)—),

5.24 and 5.38 (d, 1H, olefinic proton), 5.63 to 5.72 (m, 2H, olefinic proton) and 6.60 to 7.18 (m, 9H, aromatic, olefinic proton).

Mass spectrum (20 eV, Direct) m/e:

382 ($M^{+}$), 271 [M—OC(=O)CH=CH–CH=$CHCH_3$]$^+$, 226 (base peak),

199 [M—C(=O)O$CH_2CH_2$OC(=O)CH=CH—CH=$CHCH_3$]$^+$, 139 [—$CH_2CH_2$OC(=O)CH=CH—CH=$CHCH_3$]$^+$,

95 [—COCH=CH—CH—$CHCH_3$]$^+$ and 67 [—CH=CH—CH=$CHCH_3$]$^+$.

Infrared absorption spectrum (ν $cm.^{-1}$):

3100 to 2850 (aromatic, alkyl ν C—H), 1745, 1710 (ester ν C=O) and 1655 (olefinic ν C=C).

Example 29

[2-(Acetoxy)ethyl 2-(2-fluoro-4-biphenylyl)propionate (Compound No. 17)]

In 35 ml. of anhydrous pyridine was dissolved 7.55 g. (26.2 mmoles) of 2-hydroxyethyl-2-(2-fluoro-4-biphenylyl)propionate. To the resulting solution was added dropwise 4 g. (39.3 mmoles) of acetic anhydride with ice-cooling. After completion of the addition, the resulting reaction mixture was stirred at 40°C. for 3 hours, and then cooled. The reaction mixture was poured into water added with ice and extracted with 400 ml. of diethyl ether. The resulting extract was washed successively with 10% hydrochloride and water, and the organic layer was dried with anhydrous magnesium sulfate. After distilling away the solvent under reduced pressure, the obtained clear oily material was distilled under reduced pressure for purification to give 7.61 g. (yield: 88%) of the desired compound being a clear oily material.

The physicochemical properties of the obtained compound were consistent with those obtained in Example 23.

Example 30

[2-(Acetoxy)ethyl 2-(2-fluoro-4-biphenylyl)propionate (Compound No. 17)]

To 100 ml. of anhydrous acetonitrile were added 2.82 g. (10 mmoles) of potassium salt of FP, 0.1 g. of 18-crown-6 and 1.22 g. (10 mmoles) of 2-acetoxyethyl chloride, and the reaction mixture was stirred at a room temperature for 8 hours. The resulting reaction mixture was treated by a conventional method and purified in the same manner as in example 23 to give 1.48 g. (yield: 44.8%) of the desired compound being a clear oily material.

The physicochemical properties of the obtained compound were consistent with those obtained in Example 23.

Example 31

[2-(Propionyloxy)ethyl 2-(2-fluoro-4-biphenylyl)propionate (Compound No. 18)]

To 100 ml. of anhydrous acetonitrile were added 2.82 g. (10 mmoles) of potassium salt of FP, 1.4 g. (10 mmoles) of 2-propionyloxyethyl chloride, 1 g. of triethylamine and 1.6 g. of potassium iodide, and then the reaction mixture was stirred at 60°C. for 5 hours. The resulting reaction mixture was post-treated by a conventional method, and distilled under reduced pressure for purification to give 2.86 g. (yield: 83%) of the desired compound being a clear oily material.

The physicochemical properties of the obtained compound were consistent with those obtained in Example 24.

Example 32

[3,3-Dimethyl-2-[2-(2-fluoro-4-biphenylyl)propionyloxy]-γ-butyrolactone (Compound No. 23)]

In 40 ml. of anhydrous dichloromethane were dissolved 2.44 g. (10 mmoles) of FP, 1.3 g. (10 mmoles) of 2-hydroxy-3,3-dimethylbutyrolactone and 0.1 g. of p-dimethylaminopyridine. To the resulting solution was added dropwise 2.0 g. (10 mmoles) of dicyclohexylcarbodiimide, and the resulting reaction mixture was stirred at a room temperature for one hour. After completion of the reaction, the reaction mixture was cooled to a temperature of 0°C., and the precipitated dicyclohexylurea was filtered off. The resulting filtrate was washed successively with 0.1 N hydrochloride, a saturated sodium bicarbonate solution and a saturated sodium chloride solution, and then the organic layer was dried with anhydrous magnesium sulfate. After distilling away the solvent under reduced pressure, the resulting light yellowish liquor was

distilled under reduced pressure for purification to give 2.41 g. (yield: 68%) of the desired compound being an oily material having a boiling point of 198° to 209°C./0.9 mmHg.

Elementary analysis $C_{21}H_{21}FO_4$ (MW; 356):

Calcd. (%): C 70.79 H 5.90

Found (%): C 70.93 H 5.92.

Nuclear magnetic resonance spectrum (in $CCl_4$, TMS), δ (ppm):

0.86 to 1.17 (s × 4, 6H, lactone $CH_3$ × 2), 1.54 to 1.67 (d × 2, 3H,

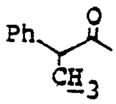), 3.83 to 3.96 (m, 3H, PhC<u>H</u>COO, —$CO_2C\underline{H_2}$—C—),

5.26 to 5.30 (s × 2, 1H, —COOC<u>H</u>—COO) and 7.08 to 7.43 (m, 8H, aromatic H).

Mass spectrum (20 eV, Direct) m/e:

356 ($M^{\dot{+}}$), 243 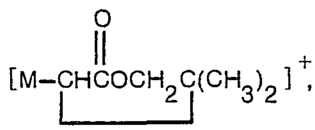,

226 [M—OCHCOCH$_2$C($CH_3$)$_2$]$^+$ and 199 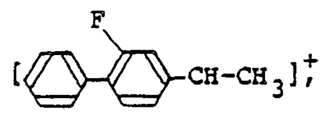

Infrared absorption spectrum (ν cm.$^{-1}$):

3100 to 2900 (aromatic, alkyl ν C—H), 1760 (ν C=O ester) and 1800 (γ-lactone ν C=O).

Refractive index: $n_D^{24.5}$ = 1.5485.

Ultraviolet absorption: λ max = 247 nm.

### Example 33

[3,3-Dimethyl-2-[2-(2-fluoro-4-biphenylyl)propionyloxy]-γ-butyrolactone (Compound No. 23)]

In 50 ml. of anhydrous tetrahydrofuran were dissolved 2.44 g. (10 mmoles) of FFP, 1.3 g. (10 mmoles) of D,L-pantoyllactone and 2.6 g. of triphenylphosphin. After adding dropwise 1.7 g. (10 mmoles) of azo-carboxylic acid diethyl ester with stirring at 0°C., the reaction was carried out at 0°C. for one hour, and then at a room temperature for one hour. After adding 20 ml. of ether to the reaction mixture, the resulting precipitate was filtered off. The resulting organic layer was washed three times with water, and then dried with anhydrous magnesium sulfate. After distilling away the solvent, the obtained residue was distilled under reduced pressure for purification to give 2.3 g. (yield: 65%) of the desired compound being a clear oily material.

The physicochemical properties of the obtained compound were consistent with those obtained in Example 32.

### Example 34

[3,3-Dimethyl-2-[2-(2-fluoro-4-biphenylyl)-propionyloxy]-γ-butyrolactone (compound 23)]

In 10 ml. of anhydrous DMF was dissolved 2.44 g. (10 mmoles) of FP in an atmosphere replaced with argon gas. After adding dropwise N,N'-carbonyldiimidazole dissolved in anhydrous DMF at a room temperature, the reaction was carried out for one hour with stirring. To the resulting reaction mixture was added dropwise 1.3 g. (10 mmoles) of D,L-pantolactone with ice-cooling. After completion of the addition, the reaction was carried out at a room temperature for 3 hours with stirring. The resulting reaction mixture was concentrated under reduced pressure, and then the residue was dissolved in ether. The resulting mixture was washed successively with water, 10% citric acid, water, a saturated sodium bicarbonate solution and a saturated sodium chloride solution, and then the organic layer was dried with anhydrous magnesium sulfate. After distilling away the solvent under reduced presssure, the obtained residue was distilled under reduced pressure for purification to give 2.5 g. (yield: 71%) of the desired compound being a clear oily material.

The physicochemical properties of the obtained compound were consistent with those obtained in Example 32.

### Example 35

[Tablet]

To 100 mg. of 1-(acetoxy)ethyl 2-(2-fluoro-4-biphenylyl)propionate was added 30 mg. of Aerosil (registered trademark, made by Japan Aerosil Co., Ltd.)., and the mixture was pulverized. To the resulting

powder were added 35 mg. of dibasic calcium phosphate anhydrous, 45 mg. of Avicel (registered trademark, made by Asahi Kassei Co., ltd.). 6 mg. of ECG 505 (carboxymethyl celllulose calcium salt made by Nichirin Chemical Industry Co., Ltd.) and 4 mg. of calcium stearate, and then the mixture was blended and compressed to give a tablet.

Example 36

[Suppository]

A mixture of 1240 mg. of Witepsol H—15 (mixture of triglyceride and monoglyceride made by Dinamit Novel Co., Ltd., Witepsol; registered trademark) and 310 mg. of Witepsol E—85 (made by Dinamit Novel Co., Ltd.) was melted at 60° to 70°C. After cooling the mixture to a temperature of 45°C., 150 mg. of 1-(acetoxy)ethyl 2-(2-fluoro-4-biphenylyl) propionate was added thereto. The resulting mixture was stirred until it become homogeneous, and then 1.7 g. thereof was injected in a container of 1.9 ml. at 40°C. to make solid with cooling.

Example 37

[Soft gelatin capsule]

(a) For oral administration

In 100 mg. of PEG 400 (polyethyleneglycol) was dissolved 100 mg. of 1-(acetoxy)ethyl 2-(2-fluoro-4-biphenylyl)propionate, and then 200 mg. of the resulting solution was filled up in a soft gelatin capsule (made by R. P. Scherer Co., Ltd.; size 3 to 2 round A).

(b) For Supporisitories

In 260 mg. of PEG 400 was dissolved 150 mg. of 1-(acetoxy)ethyl 2-(2-fluoro-4-biphenylyl)propionate, and then 410 mg. of the resulting solution was filled up in a soft gelatin capsule (made by R. P. Scherer Co., Ltd.; size: 85 to 86 suppository A).

Example 38

[Cream]

According to the following formulation, a 1% gel cream was prepared as follows:

| | |
|---|---|
| 1-(Acetoxy)ethyl 2-(2-fluoro-4-biphenylyl) propionate | 10 g. |
| Myristin isopropyl (made by Nikko Chemicals Co., Ltd.) | 100 g. |
| Ethanol | 50 g. |
| Polyoxyethylene monostearate | 10 g. |
| Carboxyvinyl polymer-940 | 15 g. |
| Coconut oil (fatty acid diethanol amide) | 30 g. |
| Distilled water | Sufficient amount |
| | Total 1000 g. |

In myristin isopropyl was dissolved 1-(acetoxy)ethyl 2-(2-fluoro-4-biphenylyl)propionate. To the resulting solution were added ethanol, carboxyvinyl polymer-940 swelled in 500 ml. of water and polyoxyethylene dissolved in 100 ml. of water, and then the resulting mixture was thoroughly stirred until it become homogeneous. To the homogeneous mixture were added coconut oil blended in 100 ml. of water and sufficient amount of distilled water, and then the resulting mixture was thoroughly stirred until it become homogeneous.

Example 39

[Inhibitory effect on carrageenan-induced edema]

With respect to the present FP derivatives (Compound Nos. 1 to 23), there was tested inhibitory effect on carrageenan-induced edema.

Five Wistar male rates weighing about 150 g. were used as one group. A 1% dispersion of carrageenan was injected intracutaneously into the foot pad of the right hind foot in a dose of 0.1 ml./rat. The compounds to be tested were orally administered to the rats fasted for 15 hours one hour before the carrageenan injection, or intraveneously administered 2 hours after the carrageenan injection. The volume of the foot subjected to the injection was measured by a mercury plethysmography 3 hours after the carrageenan injection on the test by oral route or 2 hours after the carrageenan injection on the test by intravenous route, an dthe carrageenan-induced edema was estimated from the obtained measurements. The results are shown in Table 2.

TABLE 2

| | Compound No. | $ED_{50}$ mg./kg. p.o. | $ED_{50}$ mg./kg. i.v. |
|---|---|---|---|
| Compounds of the invention | 1 | 0.5 | 0.08 |
| | 2 | 0.5 | 0.07 |
| | 3 | 0.6 | 0.10 |
| | 4 | 0.6 | 0.08 |
| | 5 | 0.5 | 0.06 |
| | 6 | 1.5 | 0.50 |
| | 7 | 0.6 | 0.10 |
| | 8 | 0.5 | 0.05 |
| | 9 | 0.7 | 0.06 |
| | 10 | 1.5 | 0.1 |
| | 11 | 2.5 | 0.6 |
| | 12 | >5.0 | >5.0 |
| | 13 | 1.5 | 0.01 |
| | 14 | 0.9 | 0.08 |
| | 15 | 2.5 | 0.3 |
| | 16 | 2.0 | 0.3 |
| | 17 | 0.7 | — |
| | 18 | 0.7 | — |
| | 19 | 0.9 | — |
| | 20 | 1.0 | — |
| | 21 | 0.9 | — |
| | 21 | 0.9 | — |
| | 23 | 0.5 | 0.05 |
| Comparative compounds | acemethacin | 8.3 | — |
| | indomethacin | 5.5 | 1.25 |
| | FP | 0.8 | 0.3 |

Example 40

[Gastric ulceration]

With respect to the present FP derivatives (Compound Nos. 1 to 23), there was tested gastric ulceration.

Five Wistar male rates weighing about 150 g. were used as one group. The rats were fasted for 24 hours before the test.

Six hours after orally administration of the present compounds to be tested, there was observed an existence of gastric ulcer by the method of Okabe et al (Ohyoyokuri, *16*, 241 to 247 (1978). The $UD_{50}$ values were calculated from the ulcer incidence of gastric ulcer by the method of Litchfiled-Wilcoxon. The results are shown in Table 3.

TABLE 3

| | Compound No. | $UD_{50}$ mg./kg. |
|---|---|---|
| Compounds of the invention | 1 | 3.6 |
| | 2 | 2.5 |
| | 3 | 2.0 |
| | 4 | 2.0 |
| | 5 | 2.0 |
| | 6 | 5.0 |
| | 7 | 2.5 |
| | 8 | 2.5 |
| | 9 | 3.0 |
| | 10 | 5.0 |
| | 11 | 10.0 |
| | 12 | >10.0 |
| | 13 | 5.0 |
| | 14 | 4.0 |
| | 15 | 7.0 |
| | 16 | 7.0 |
| | 17 | 2.0 |
| | 18 | 2.0 |
| | 19 | 3.0 |
| | 20 | 3.0 |
| | 21 | 3.0 |
| | 22 | 3.0 |
| | 23 | 2.5 |
| Comparative compounds | acemethacin | 17.5 |
| | indomethacin | 5.0 |
| | FP | 1.0 |

Example 41

[Hydrolysis of FR derivatives in plasma]

With respect to the present FP derivatives (Compound Nos. 1 to 23), there was tested the hydrolysis rate in human plasma or rat plasma.

The compounds to be tested (Compound Nos. 1 to 16) and the compounds to be tested (Compound Nos. 17 to 23) were, respectively, added to 1 ml. of human plasma and 1 ml. of rat plasma, in an amount corresponding to 50 µg. of FP. Each reaction mixture was incubated at 37°C. for one hour.

The free FP formed by esterase in the plasma was extracted with benzene. After treating the extract with N,O-bis(trimethylsilylacetamide) for trimethylsilylation, the obtained material was determined by gas-liquid chromatography. The results are shown in Table 4.

TABLE 4

| | Compound No. | Hydrolyzing rate (%) |
|---|---|---|
| Compounds of the invention | 1 | 97.5 |
| | 2 | 94.6 |
| | 3 | 86.3 |
| | 4 | 81.4 |
| | 5 | 114.8 |
| | 6 | 3.8 |
| | 7 | 70.3 |
| | 8 | 70.7 |
| | 9 | 56.3 |
| | 10 | 35.2 |
| | 11 | 8.3 |
| | 12 | 2.0 |
| | 13 | 36.0 |
| | 14 | 42.0 |
| | 15 | 19.8 |
| | 16 | 20.4 |
| | 17* | 100.9 |
| | 18* | 98.9 |
| | 19* | 99.5 |
| | 20* | 99.8 |
| | 21* | 100.3 |
| | 22* | 101.0 |
| | 23* | 72.3 |
| Comparative compounds | acemethacin | 4.2 |
| | indomethacin | — |
| | FP | — |

* Values in rat plasma.

Example 42

[Acute toxicity]

Eight male SLC-ddY mice 5 week old weighing 25 to 30 g. were used as one group. The compound to be tested was orally administered using a stomach tube. The animals were kept under observation for 2 weeks. The numbers of dead animals were counted and the $LD_{50}$ values were calculated by the method of Litchfield Wilconxon. The resutls are shown in Table 5.

TABLE 5

| | Compound No. | $LD_{50}$ mg./kg. |
|---|---|---|
| Compounds of the invention | 1 | 880 |
| | 2 | 750 |
| | 3 | 600 |
| | 4 | 600 |
| | 5 | 900 |
| | 6 | >1000 |
| | 7 | 840 |
| | 8 | 650 |
| | 9 | 800 |
| | 10 | 880 |
| | 11 | >1000 |
| | 12 | >1000 |
| | 13 | 900 |
| | 14 | 800 |
| | 15 | 1000 |
| | 16 | 1000 |
| | 17 | 650 |
| | 18 | 700 |
| | 19 | 800 |
| | 20 | 880 |
| | 21 | 750 |
| | 22 | 750 |
| | 23 | 670 |
| Comparative compounds | acemethacin | 18.0 |
| | indomethacin | 14.0 |
| | FP | 440 |

**Claims for the Contracting States: BE CH DE FR GB IT LI LU NL**

1. A biphenylylpropionic acid derivative having the formula (I):

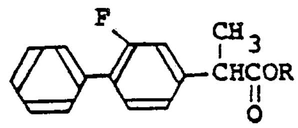

(I)

wherein R is

a) an alkylcarbonyloxyalkyl group or an alkenylcarbonyloxyalkyl group having the formula (II):

$$-\overset{\overset{\displaystyle R^1}{|}}{C}H-(CH_2)_m-O\overset{\overset{\displaystyle O}{\|}}{C}R^2 \qquad (II)$$

wherein $R^1$ is hydrogen or a lower alkyl group having 1 to 5 carbon atoms, $R^2$ is an alkyl group having 1 to 15 carbon atoms or an alkenyl group having 2 to 8 carbon atoms, and m is 0 or an integer of 1, provided that when $R^1$ is a lower alkyl group, m is 0, or

b) a lactone having the formula (III):

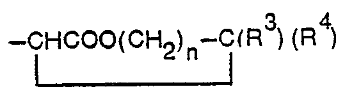

(III)

wherein $R^3$ and $R^4$ are hydrogen atom or a lower alkyl group having 1 to 2 carbon atoms, and n is an integer of 1 or 2.

2. The biphenylylpropionic acid derivative of Claim 1, wherein R is acetoxymethyl, propionyloxymethyl, isobutyryloxymethyl, privaloyloxymethyl, palmitoyloxymethyl, crotonoyloxymethyl, 3,3-dimethylacryloyloxymethyl, 1-acetoxyethyl, 1-acetoxypropyl, 1-propionyloxyethyl, 1-isobutyryloxyethyl, 1-pivaloxyloxyethyl, 1-palmitoyloxyethyl, 1-crotonoyloxyethyl, 1-(3,3-dimethylacryloyloxy)-ethyl, 1-(2,4-hexadienoyloxy)ethyl, 2-acetoxyethyl, 2-propionyloxyethyl, 2-crotonoyloxyethyl, 2-(3,3-dimethylacryloyloxy)ethyl, 2-(2,4-hexadienoyloxy)ethyl, 2-(3,7-dimethyl-2,6-octadienoyloxy)ethyl or 3,3-dimethyl-γ-butyrolactone-2-yl group.

3. A process for preparing a biphenylylpropionic acid derivative having the formula (I):

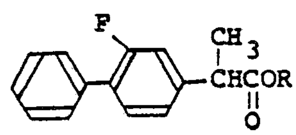

(I)

wherein R is

a) an alkylcarbonyloxyalkyl group of an alkenylcarbonyloxyalkyl group having the formula (II):

$$-\overset{\overset{\displaystyle R^1}{|}}{C}H-(CH_2)_m-O\overset{\overset{\displaystyle O}{\|}}{C}R^2 \qquad (II)$$

wherein $R^1$ is hydrogen atom or a lower alkyl group having 1 to 5 carbon atoms, $R^2$ is an alkyl group having 1 to 15 carbon atoms or an alkenyl group having 2 to 8 carbon atoms, m is 0 or an integer of 1, provided that when $R^1$ is a lower alkyl group, m is 0, or

(b) a lactone having the formula (III):

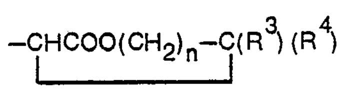

(III)

wherein $R^3$ and $R^4$ are hydrogen atom or a lower alkyl group having 1 to 2 carbon atoms; n is an integer of 1 or 2, which comprises reacting 2-(2-fluoro-4-biphenylyl)propionic acid or the salt thereof having the formula (IV):

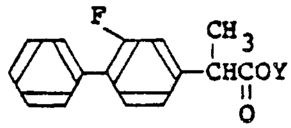

(IV)

wherein Y is hydrogen atom or a metal cation, with a compound having the formula (V):

$$X-\underset{\displaystyle |}{\overset{\displaystyle R^1}{C}}H-(CH_2)_m-O\overset{\displaystyle O}{\overset{\|}{C}}R^2 \qquad \text{(II)}$$

wherein $R^1$, $R^2$ and m are as defined above and X is a halogen atom, or a compound having the formula (VI):

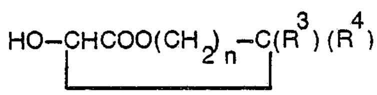

(VI)

wherein $R^3$, $R^4$ and n are as defined above.

4. A process for preparing a biphenylylpropionic acid derivative having the formula ($I_0$):

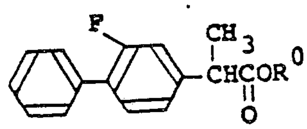

($I_0$)

wherein $R^0$ is an alkylcarbonyloxyalkyl group or an akenylcarbonyloxyalkyl group having the formula ($II_0$):

$$-CH_2-(CH_2)-O\overset{\displaystyle O}{\overset{\|}{C}}R^2 \qquad (II_0)$$

wherein $R^2$ is an alkyl group havng 1 to 15 carbon atoms or an alkenyl group having 2 to 8 carbon atoms, which comprises reacting biphenylylpropionic acid 2-hydroxyethyl ester having the formula (VII):

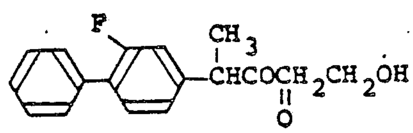

(VII)

with an acid anhydride having the formula (VIII):

$$(R^5\underset{\underset{\displaystyle O}{\|}}{C})_2O \qquad \text{(VIII)}$$

wherein $R^5$ is a lower alkyl group having 1 to 2 carbon atoms or an alkenyl group having 2 to 7 carbon atoms.

5. A process for preparing a biphenylylpropionic acid derivative having the formula ($I_0$):

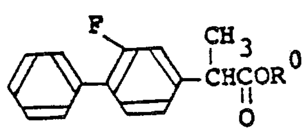

($I_0$)

wherein $R^0$ is an alkylcarbonyloxyalkyl group or an alkenylcarbonyloxyalkyl group having the formula (II):

$$-CH_2-(CH_2)_m-O\overset{\displaystyle O}{\overset{\|}{C}}R^2 \qquad \text{(II)}$$

wherein $R^2$ is an alkyl group having 1 to 15 carbon atoms or an alkenyl group having 2 to 8 carbon atoms, which comprises reacting a biphenylylpropionic acid 2-haloethyl ester having the formula (IX)

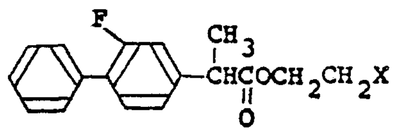

(IX)

wherein X is a halogen atom, with an acid having the formula (X):

$$R^5COOH \qquad (X)$$

wherein $R^5$ is a lower alkyl group having 1 to 2 carbon atoms or an alkenyl group having 2 to 7 carbon atoms.

6. A pharmaceutical composition having antiinflammatory, analgesic and antipyretic activities which comprises, as the effective ingredient, an effective amount of a biphenylylpropionic acid derivative having the formula (I):

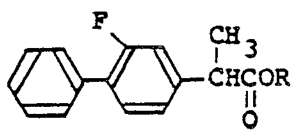

(I)

wherein R is

a) an alkylcarbonyloxyalkyl group or an alkenylcarbonyloxyalkyl group having the formula (II):

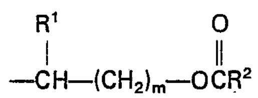

(II)

wherein $R^1$ is hydrogen atom or a lower alkyl group having 1 to 5 carbon atoms, $R^2$ is an alkyl group having 1 to 15 carbon atoms or an alkenyl group having 2 to 8 carbon atoms, m is 0 or an integer of 1, provided that when $R^1$ is a lwoer alkyl group, m is 0, or

b) a lactone having the formula (III):

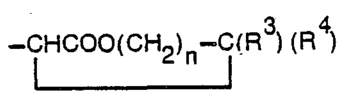

(III)

wherein $R^3$ and $R^4$ are hydrogen atom or a lower alkyl group having 1 to 2 carbon atoms, and n is an integer of 1 or 2 and a pharmaceutically acceptable carrier.

7. The pharmaceutical composition of Claim 6 wherein R is acetoxymethyl, propionyloxymethyl, isobutyryloxymethyl, pivaloyloxymethyl, palmitoyloxymethyl, crotonoyloxymethyl, 3,3-dimethylacryloyloxymethyl, 1-acetoxyethyl, 1-acetoxypropyl, 1-propionyloxyethyl, 1-isobutyryloxyethyl, 1-pivaloyloxyethyl, 1-palmitoyloxyethyl, 1-crotonoyloxyethyl, 1-(3,3-dimethylacryloyloxy)ethyl, 1-(2,4-hexadienoyloxy)ethyl, 2-acetoxyethyl, 2-propionyloxyethyl, 2-crotonoyloxyethyl, 2-(3,3-dimethylacryloyloxy)ethyl, 2-(2,4-hexadienoyloxy)ethyl, 2-(3,7-dimethyl-2,6-octadienoyloxy)ethyl or 3,3-dimethyl-γ-butyrolactone-2-yl group.

8. The pharmaceutical composition of Claim 6, which is in a preparation form of tablet, capsule, suppository and cream.

**Claim for the contracting state: AT**

A process for preparing a biphenylylpropionic acid derivative having the formula (I):

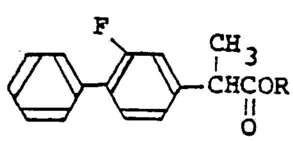

(I)

wherein R is

a) an alkylcarbonyloxyalkyl group or an alkenylcarbonyloxyalkyl group having the formula (II):

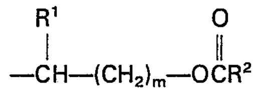

(II)

wherein $R^1$ is hydrogen or a lower alkyl group having 1 to 5 carbon atoms, $R^2$ is an alkyl group having 1 to 15 carbon atoms or an alkenyl group having 2 to 8 carbon atoms, and m is 0 or an integer of 1, provided that when $R^1$ is a lower alkyl group, m is 0, or

b) a lactone having the formula (III):

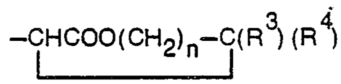

(III)

wherein $R^3$ and $R^4$ are hydrogen atom or a lower alkyl group having 1 to 2 carbon atoms; n is an integer of 1 or 2, which comprises reacting 2-(2-fluoro-4-biphenylyl)propionic acid or the salt thereof having the formula (IV):

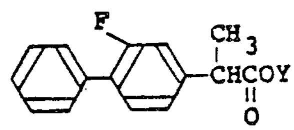

(IV)

wherein Y is hydrogen atom or a metal cation, with a compound having the formula (V):

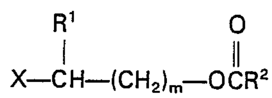

(V)

wherein $R^1$, $R^2$ and m are as defined above and X is a halogen atom, or a compound having the formula (VI):

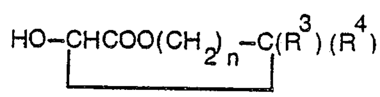

(VI)

wherein $R^3$, $R^4$ and n are as defined above.

**Patentansprüche für die Vertragstaaten: BE CH DE FR GB IT LI LU NL**

1. Biphenylylpropionsäurederivat der Formel (I):

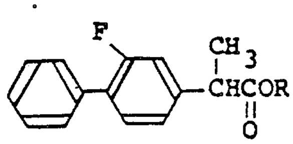

(I)

worin R bedeutet:

a) eine Alkylcarbonyloxyalkylgruppe oder eine Alkenylcarbonyloxyalkylgruppe der Formel (II):

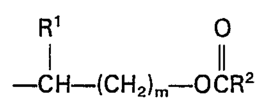

(II)

worin $R^1$ ein Wasserstoffatom oder eine niedere Alkylgruppe mit 1 bis 5 Kohlenstoffatomen, $R^2$ eine Alkylgruppe mit 1 bis 15 Kohlenstoffatomen oder eine Alkenylgruppe mit 2 bis 8 Kohlenstoffatomen und m die Zahl 0 oder die ganze Zahl 1 bedeutet, mit der Maßgabe, daß dann, wenn $R^1$ eine niedere Alkylgruppe bedeutet, m = 0, oder

b) ein Lacton der Formel (III):

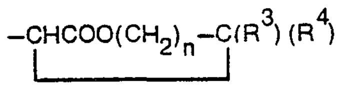

(III)

worin $R^3$ und $R^4$ ein Wasserstoffatom oder eine niedere Alkylgruppe mit 1 bis 2 Kohlenstoffatomen und n die ganze Zahl 1 oder 2 bedeuten.

2. Biphenylylpropionsäurederivat nach Anspruch 1, worin R bedeutet eine Acetoxymethyl-, Propionyloxymethyl-, Isobutyryloxymethyl-, Pivaloyloxymethyl-, Palmitoyloxymethyl-, Crotonoyloxymethyl-, 3,3-Dimethylacryloyloxymethyl-, 1-Acetoxyethyl-, 1-Acetoxypropyl-, 1-Propionyloxyethyl-, 1-Isobutyryloxyethyl-, 1-Pivaloyloxyethyl-, 1-Palmitoyloxyethyl-, 1-Crotonoyloxyethyl-, 1-(3,3-Dimethylacryloyloxy)ethyl-, 1-(2,4-Hexadienoyloxy)ethyl-, 2-Acetoxyethyl-, 2-Propionyloxyethyl-, 2-Crotonoyloxyethyl-, 2-(3,3-Dimethylacryloyloxy)ethyl-, 2-(2,4-Hexadienoyloxy)ethyl-, 2-(3,7-Dimethyl-2,6-octadienoyloxy)ethyl- oder 3,3-Dimethyl-γ-butyrolacton-2-yl-Gruppe.

3. Verfahren zur Herstellung eines Biphenylylpropionsäurederivats der Formel (I):

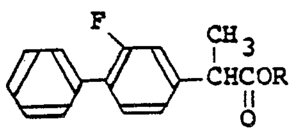

(I)

worin R bedeutet:

a) eine Alkylcarbonyloxyalkylgruppe oder eine Alkenylcarbonyloxyalkylgruppe der Formel (II):

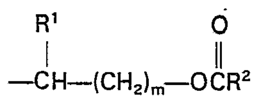

(II)

worin $R^1$ ein Wasserstoffatom oder eine niedere Alkylgruppe mit 1 bis 5 Kohlenstoffatomen, $R^2$ eine Alkylgruppe mit 1 bis 15 Kohlenstoffatomen oder eine Alkenylgruppe mit 2 bis 8 Kohlenstoffatomen, m die Zahl 0 oder die ganze Zahl 1 bedeutet, mit der Maßgabe, daß dann, wenn $R^1$ eine niedere Alkylgruppe bedeutet, m = 0, oder

b) ein Lacton der Formel (III):

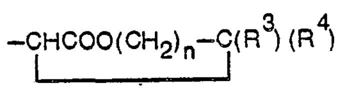

(III)

worin $R^3$ und $R^4$ ein Wasserstoffatom oder eine niedere Alkylgruppe mit 1 bis 2 Kohlenstoffatomen; n die ganze Zahl 1 oder 2 bedeuten, das umfaßt die Umsetzung von 2-(2-Fluoro-4-biphenylyl)-propionsäure oder ihres Salzes der Formel (IV):

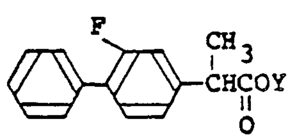

(IV)

worin Y ein Wasserstoffatom oder ein Metallkation bedeutet, mit einer Verbindung der Formel (V):

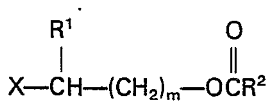

(V)

worin $R^1$, $R^2$ und m wie oben definiert sind und X ein Halogenatom bedeutet, oder mit einer Verbindung der Formel (VI):

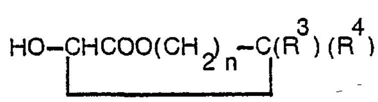

(VI)

worin $R^3$, $R^4$ und n wie oben definiert sind.

4. Verfahren zur Herstellung eines Biphenylylpropionsäurederivats der Formel ($I_0$):

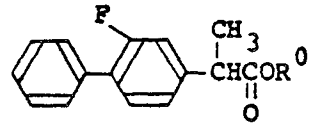

($I_0$)

worin $R^0$ ein Alkylcarbonyloxyalkylgruppe oder eine Alkenylcarbonyloxyalkylgruppe der Formel ($II_0$) bedeutet:

$$-CH_2-(CH_2)-O\overset{O}{\overset{\|}{C}}R^2 \qquad (II_0)$$

worin $R^2$ eine Alkylgruppe mit 1 bis 15 Kohlenstoffatomen oder eine Alkenylgruppe mit 2 bis 8 Kohlenstoffatomen bedeutet, das umfaßt die Umsetzung von Biphenylylpropionsäure-2-hydroxyethylester der Formel (VII):

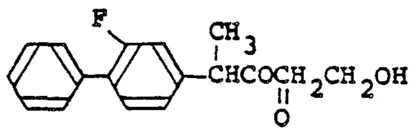

(VII)

mit einem Säureanhydrid der Formel (VIII):

$$(R^5\overset{O}{\overset{\|}{C}})_2O \qquad (VIII)$$

worin $R^5$ eine niedere Alkylgruppe mit 1 bis 2 Kohlenstoffatomen oder ein Alkenylgruppe mit 2 bis 7 Kohlenstoffatomen bedeutet.

5. Verfahren zur Herstellung eines Biphenylylpropionsäurederivats der Formel ($I_0$):

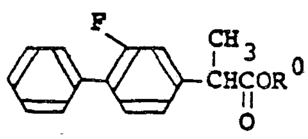

($I_0$)

worin $R^0$ eine Alkylcarbonyloxyalkylgruppe oder eine Alkenylcarbonyloxyalkylgruppe der Formel (II) bedeutet:

$$-\overset{R^1}{\overset{|}{C}}H_2-(CH_2)_m-O\overset{O}{\overset{\|}{C}}R^2 \qquad (II)$$

worin $R^2$ eine Alkylgruppe mit 1 bis 15 Kohlenstoffatomen oder eine Alkenylgruppe mit 2 bis 8 Kohlenstoffatomen bedeutet, das umfaßt die Umsetzung eines Biphenylylpropionsäure-2-halogenethylesters der Formel (IX):

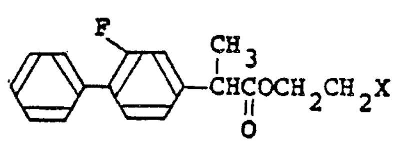

(IX)

worin X ein Halogenatom bedeutet, mit einer Säure der Formel (X):

$$R^5COOH \qquad (X)$$

worin $R^5$ eine niedere Alkylgruppe mit 1 bis 2 Kohlenstoffatomen oder eine Alkenylgruppe mit 2 bis 7 Kohlenstoffatomen bedeutet.

6. Pharmazeutische Zusammensetzung mit antiinflammatorischen, analgetischen und antipyretischen Aktivitäten, die als wirksamen Bestandteil (Wirkstoff) eine wirksame Menge eines Biphenylylpropionsäurederivats der Formel (I):

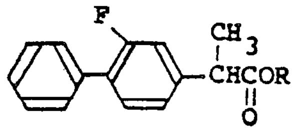

(I.)

worin R bedeutet:

a) eine Alkylcarbonyloxyalkylgruppe oder eine Alkenylcarbonyloxyalkylgruppe der Formel (II):

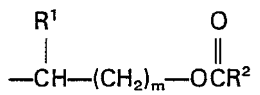

(II)

worin $R^1$ ein Wasserstoffatom oder eine niedere Alkylgruppe mit 1 bis 5 Kohlenstoffatomen, $R^2$ eine Alkylgruppe mit 1 bis 15 Kohlenstoffatomen oder eine Alkenylgruppe mit 2 bis 8 Kohlenstoffatomen, m die Zahl 0 oder die ganze Zahl 1 bedeutet, mit der Maßgabe, daß dann, wenn $R^1$ eine niedere Alkylgruppe bedeutet, m = 0, oder

b) ein Lacton der Formel (III):

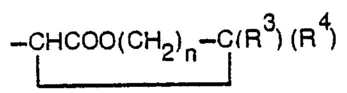

(III)

worin $R^3$ und $R^4$ ein Wasserstoffatom oder eine niedere Alkylgruppe mit 1 bis 2 Kohlenstoffatomen und n die ganze Zahl 1 oder 2 bedeuten, und einen pharmazeutisch akzeptablen Träger enthält.

7. Pharmazeutische Zusammensetzung nach Anspruch 6, worin R bedeutet eine Acetoxymethyl-, Propionyloxymethyl-, Isobutyryloxymethyl-, Pivaloyloxymethyl-, Palmitoyloxymethyl-, Crotonoyloxymethyl-, 3,3-Dimethylacryloyloxymethyl-, 1-Acetoxyethyl-, 1-Acetoxypropyl-, 1-Propionyloxyethyl-, 1-Isobutyryloxyethyl-, 1-Pivaloyloxyethyl-, 1-Palmitoyloxyethyl-, 1-Crotonoyloxyethyl-, 1-(3,3-Dimethylacryloyloxy)ethyl-, 1-(2,4-Hexadienoyloxy)ethyl-, 2-Acetoxyethyl-, 2-Propionyloxyethyl-, 2-Crotonoyloxyethyl-, 2-(3,3-Dimethylacryloyloxy)ethyl-, 2-(2,4-Hexadienoyloxy)ethyl-, 2-(3,7-Dimethyl-2,6-octadienoyloxy)ethyl- oder 3,3-Dimethyl-γ-butyrolacton-2-yl-Gruppe.

8. Pharmazeutische Zusammensetzung nach Anspruch 6, die als Präparat in Form einer Tablette, Kapsel, eines Suppositoriums und einer Creme vorliegt.

**Patentanspruch für den Vertragsstaat: AT**

Verfahren zur Herstellung eines Biphenylylpropionsäurederivats der Formel (I):

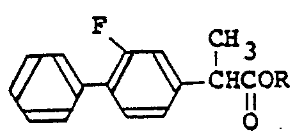

(I.)

worin R bedeutet:

a) eine Alkylcarbonyloxyalkylgruppe oder eine Alkenylcarbonyloxyalkylgruppe der Formel (II):

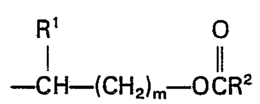

(II)

worin $R^1$ ein Wasserstoffatom oder eine niedere Alkylgruppe mit 1 bis 5 Kohlenstoffatomen, $R^2$ eine Alkylgruppe mit 1 bis 15 Kohlenstoffatomen oder eine Alkenylgruppe mit 2 bis 8 Kohlenstoffatomen, m die Zahl 0 oder die ganze Zahl 1 bedeutet, mit der Maßgabe, daß dann, wenn $R^1$ eine niedere Alkylgruppe bedeutet, m = 0, oder

b) ein Lacton der Formel (III):

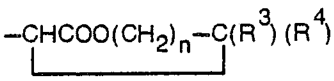

(III)

worin $R^3$ und $R^4$ ein Wasserstoffatom oder eine niedere Alkylgruppe mit 1 bis 2 Kohlenstoffatomen und n die ganze Zahl 1 oder 2 bedeuten, das umfaßt die Umsetzung von 2-(2-Fluoro-4-biphenylyl)-propionsäure oder ihres Salzes der Formel (IV):

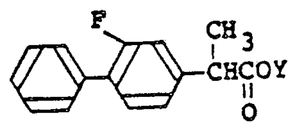

(IV)

worin Y ein Wasserstoffatom oder ein Metallkation bedeutet, mit einer Verbindung der Formel (V):

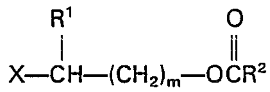

(II)

worin $R^1$, $R^2$ und m wie oben definiert sind und X ein Halogenatom bedeutet, oder mit einer Verbindung der Formel (VI):

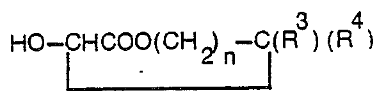

(VI)

worin $R^3$, $R^4$ und n wie oben definiert sind.

**Revendications pour les Etats contractants: BE CH DE FR GB IT LI LU NL**

1. Dérivé d'acide biphénylylpropionique répondant à la formule (I):

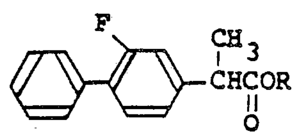

(I.)

dans laquelle R représente

(a) un groupe alkylcarbonyloxyalkyle ou un groupe alcénylcarbonyloxyalkyle répondant à la formule (II):

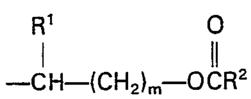

. (II)

dans laquelle $R^1$ est un atome d'hydrogène ou un radical alkyle inférieur de 1 à 5 atomes de carbone, $R^2$ est un radical alkyle de 1 à 15 atomes de carbone ou alcényle de 2 à 8 atomes de carbone et *m* est 0 ou 1, à la condition que quand $R^1$ est un radical akyle inférieur, *m* soit 0, ou

b) une lactone répondant à la formule (III):

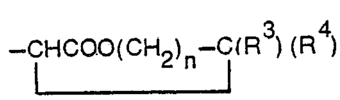

(III)

dans laquelle $R^3$ et $R^4$ représentent chacun un atome d'hydrogène ou un radical alkyle inférieur de 1 à 2 atomes de carbone et *n* est un nombre entier 1 ou 2.

2. Dérivé d'acide biphénylylpropionique selon la revendication 1, dans lequel R représente un groupe acétoxyméthyle, propionyloxyméthyle, isobutyryloxyméthyle, pivaloyloxyméthyle, palmitoyloxyméthyle, crotonoyloxyméthyle, 3,3-diméthylacryloyloxyméthyle, 1-acétoxyéthyle, 1-acétoxypropyle, 1-propionyloxyéthyle, 1-isobutyryloxyéthyle, 1-pivaloyloxyéthyle, 1-palmitoyloxyéthyle, 1-crotonoyloxyéthyle, 1-(3,3-diméthylacryloyloxy)-éthyle, 1-(2,4-hexadiènoyloxy)-éthyle, 2-acétoxyéthyle, 2-propionyloxyéthyle, 2-crotonoyloxyéthyle, 2-(3,3-diméthylacrylolyloxy)-éthyle, 2-(2,4-hexadiènoyloxy)-éthyle, 2-(3,7-diméthyl-2,6-octadiènoyloxy)éthyle ou 3,3-diméthyl-1-butyrolactone-2-yle.

3. Procédé de préparation d'un dérivé d'acid biphénylylpropionique répondant à la formule (I)

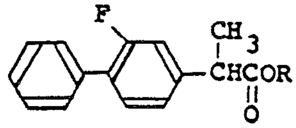 (I.)

dans laquelle R représente

(a) un groupe alkylcarbonyloxyalkyle ou alcénylcarbonyloxyalkyle répondant de formule (II):

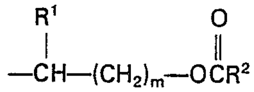 (II)

dans laquelle $R^1$ est un atome d'hydrogène ou un radical alkyle inférieur de 1 à 5 atomes de carbone, $R^2$ est un radical alkyle de 1 à 15 atomes de carbone ou alcényle de 2 à 8 atomes de carbone *m* est 0 ou un nombre entier de 1, à la condition que quand $R^1$ est un radical akyle inférieur, *m* soit 0, ou

b) une lactone de formule (III):

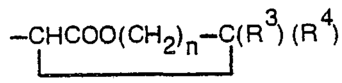 (III)

dans laquelle $R^3$ et $R^4$ représentent chacun un atome d'hydrogène ou un radical alkyle inférieur de 1 à 2 atomes de carbone; *n* est un nombre entier 1 ou 2, qui comprend le fait de faire réagir un acide 2-(2-fluoro-4-biphénylyl)propionique ou un sel de celui-ci répondant à la formule (IV):

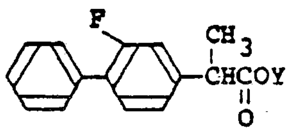 (IV)

dans laquelle Y est un atome d'hydrogène ou un cation métallique, avec un composé de formule (V):

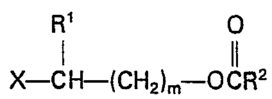 (V)

dans laquelle $R^1$, $R^2$ et *m* sont tels que définis ci-dessus et X est un atome d'halogène, ou un composé de formule (VI)

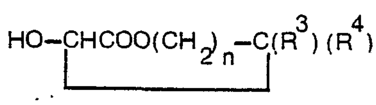 (VI)

dans laquelle $R^3$, $R^4$ et *n* sont tels que définis plus haut.

4. Procédé de préparation d'un dérivé d'acide biphénylylpropionique répondant à la formule ($I_0$):

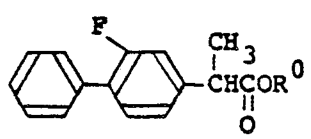 ($I_0$)

dans laquelle $R^0$ est un groupe alkylcarbonyloxyalkyle ou alcénylcarbonyloxyalkyle répondant à la formule ($II_0$):

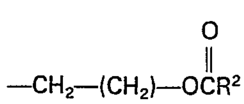 ($II_0$)

dans laquelle $R^2$ est un radical alkyle de 1 à 15 atomes de carbone ou alcényle de 2 à 8 atomes de carbone,

qui comprend le fait de faire réagir un ester 2-hydroxyéthylique d'acide biphénylylpropionique répondant à la formule (VII):

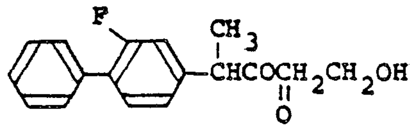

(VII)

avec un anhydride d'acide de formule (VIII):

$(R^5C)_2O$
||
O

(VIII)

dans laquelle $R^5$ est un radical alkyle inférieur de 1 à 2 atomes de carbone ou alcényle de 2 à 7 atomes de carbone.

5. Procédé de préparation d'un dérivé d'acide biphénylylpropionique de formule ($I_0$):

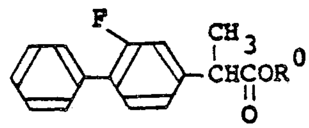

($I_0$)

dans laquelle $R^0$ est un groupe alkylcarbonyloxyalkyle ou alcénylcarbonyloxyalkyle de formule (II):

O
||
$-CH_2-(CH_2)_m-OCR^2$

(II)

dans laquelle $R^2$ est un radical alkyle de 1 à 15 atomes de carbone ou alcényle de 2 à 8 atomes de carbone, qui comprend le fait de faire réagir un ester 2-haloéthylique d'acide biphénylylpropionique de formule (IX):

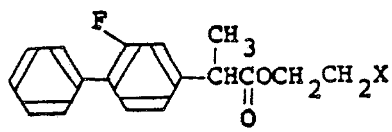

(IX)

dans laquelle X est un atome d'halogène, avec un acide de formule (X):

$R^5COOH$ (X)

dans laquelle $R^5$ est un radical alkyle inférieur de 1 à 2 atomes de carbone ou alcényle de 2 à 7 atomes de carbone.

6. Composition pharmaceutique ayant un effet anti-inflammatoire, analgésique et antipyrétique, qui comprend, à titre d'ingrédient actif, une quantité efficace d'un dérivé d'acide biphénylylpropionique répondant à la formule (I):

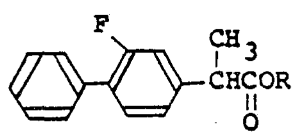

(I)

dans laquelle R représente

(a) un groupe alkylcarbonyloxyalkyle ou alcénylcarbonyloxyalkyle formule (II):

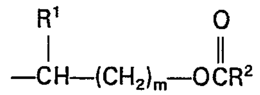

(II)

dans laquelle $R^1$ est un atome d'hydrogène ou un radical alkyle inférieur de 1 à 5 atomes de carbone, $R^2$ est un radical alkyle de 1 à 15 atomes de carbone ou alcényle de 2 à 8 atomes de carbone et *m* est 0 ou im nombre entier de 1, à la condition que quand $R^1$ est un radical akyle inférieur, *m* soit 0, ou

b) une lactone de à la formule (III):

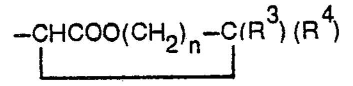

(III)

dans laquelle $R^3$ et $R^4$ représentent chacun un atome d'hydrogène ou un radical alkyle inférieur de 1 à 2 atomes de carbone et *n* est un nombre entier 1 ou 2 et un véhicule pharmaceutiquement acceptable.

7. Composition pharmaceutique selon la revendication 6, dans laquelle R représente le groupe: acétoxyméthyle, propionyloxyméthyle, isobutyryloxyméthyle, pivaloyloxyméthyle, palmitoyloxyméthyle, crotonoyloxyméthyle, 3,3-diméthylacryloyloxyméthyle, 1-acétoxyéthyle, 1-acétoxypropyle, 1-propionyloxyéthyle, 1-isobutyryloxyéthyle, 1-pivaloyloxyéthyle, 1-palmitoyloxyéthyle, 1-crotonoyloxyéthyle, 1-(3,3-diméthylacryloyloxy)éthyle, 1-(2,4-hexadiènoyloxy)-éthyle, 2-acétoxyéthyle, 2-propionyloxyéthyle, 2-crotonoyloxyéthyle, 2-(3,3-diméthylacryloyloxy)-éthyle, 2-(2,4-hexadiènoyloxy)-éthyle, 2-(3,7-diméthyl-2,6-octadiènoyloxy)éthyle ou 3,3-diméthyl-1-butyrolactone-2-yle.

8. Composition pharmaceutique selon la revendication 6, qui est sous forme de comprimés de capsules, de suppositoires ou de crèmes.

**Revendication pour l'Etat contractant: AT**

1. Procédé de préparation d'un dérivé d'acide biphénylylpropionique répondant à la formule (I):

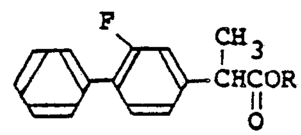

(I)

dans laquelle R est

(a) un groupe alkylcarbonyloxyalkyle ou alcénylcarbonyloxyalkyle répondant à la formule (II):

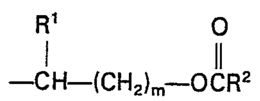

(II)

dans laquelle $R^1$ est un atome d'hydrogène ou un radical alkyle inférieur de 1 à 5 atomes de carbone, $R^2$ est un radical alkyle dę 1 à 15 atomes de carbone ou alcényle de 2 à 8 atomes de carbone et *m* est 0 ou un nombre entier de 1, à la condition que quand $R^1$ est un radical akyle inférieur, *m* soit 0, ou

b) une lactone de formule (III):

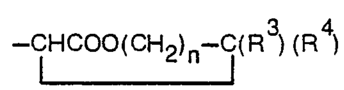

(III)

dans laquelle $R^3$ et $R^4$ représentent chacun un atome d'hydrogène ou un radical alkyle inférieur de 1 à 2 atomes de carbone; *n* est un nombre entier de 1 ou 2, qui comprend le fait de faire réagir un acide 2-(2-fluoro-4-biphénylyl)propionique ou un sel de celui-ci répondant à la formule (IV):

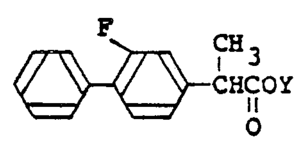

(IV)

dans laquelle Y est un atome d'hydrogène ou un cation métallique avec un composé de formule (V):

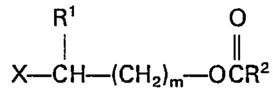

(V)

dans laquelle $R^1$, $R^2$ et *m* sont tels que définis plus haut et X est un atome d'halogène, ou un composé de formule (VI)

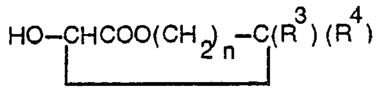

(VI)

dans laquelle $R^3$, $R^4$ et *n* sont tels que définis plus haut.